# EUROPEAN PATENT APPLICATION

(11) **EP 4 752 217 A1**
(43) Date of publication of application: **03.06.2026**
(21) Application number: 24216167.7
(22) Date of filing: 28.11.2024
(51) Int. Cl.: C12N 5/0783, A61K 35/17

(54) **MANUFACTURING METHOD FOR ACTIVATED LYMPHOCYTES**

(71) Applicant: invIOs GmbH, 1030 Wien (AT)
(72) Inventor: KUTTKE, Mario, 1110 Wien (AT)
(74) Representative: SONN Patentanwälte GmbH & Co KG

(57) **Abstract**

The invention provides an in-vitro or ex-vivo method of expanding lymphocytes and a population of expanded tumor infiltrating lymphocytes (TILs) for use in a method for treating cancer comprising providing a population of lymphocytes, wherein the lymphocytes are from a biological sample;
culturing the lymphocytes in a growth medium; expanding the lymphocytes in an expansion medium comprising nutrients and differentiation factors and/or cytokines, wherein the differentiations factors and/or cytokines comprise or consist of interleukin-2,
interleukin-7, interleukin-15, and interleukin-21; obtaining expanded lymphocytes.

## Description

The present invention relates to the field of immune cell preparation, culturing, and differentiation for adoptive cell therapy.

### Background

Over the past decade, cell-based immunotherapy has provided new and powerful strategies in solid cancer therapy.

Adoptive cell therapy (ACT) is an immunotherapy that utilizes modified or non-modified and ex vivo expanded immune cells that are reinfused intravenously into the patient for a therapy, such as cancer treatment. The predominant approach utilizes T cells from peripheral blood modified to express either a specific T cell receptor (TCR or a chimeric antigen receptor (CAR) against a tumor-associated cell surface antigen (TAA) of tumor cells). Another form of ACT uses tumor infiltrating lymphocytes (TILs) that use the patient's own immune cells from the microenvironment of the solid tumor or that are harvested directly from resected cancer tissue. FDA-approved CAR T cell therapies target B cell derived lymphomas, leukemias or myelomas. Despite ongoing research and development in academia and industry there are only two clinically approved immune cell therapies for solid tumors available yet: Lifileucel^{®}, the first cellular therapy to be approved for a solid tumor, the skin cancer melanoma and afamitresgene autoleucel (Afami-cel; Tecelra) for the treatment of MAGE-A4-positive synovial sarcoma

WO 2022/229464 A1 describes a single vessel lymphocyte expansion protocol which yields T cells of a high CD27/CD28 expression and low CD45RA/CD57/KLRG1 expression marker profile.

WO 2019/086711 A1 describes a method for expansion of antigen-specific lymphocytes in the presence of an antigen.

US 2011/268754 A1 describes a method for cancer therapy in a patient administering lymphodepleting chemotherapy and subsequently administering autologous T-cell that have been isolated and selected for the recognition of the cancer and have been expanded ex vivo.

WO 2023/237764 A1 describes an in-vitro method for transiently modifying immune cells in a closed processing system.

WO 2023/147488 A1 discloses compositions and methods for the treatment of cancers using modified TILs, wherein the modified TILs include one or more immunomodulatory agents (e.g, cytokines) associated with their cell surface.

Current protocols for expansion of TILs typically involve two main amplification processes. An initial TIL culture involves the incubation of tumor samples in a culture medium enriched with interleukin-2 (IL-2) to obtain an initial bulk amount of TILs called pre-rapid expansion procedure (pre-REP). TILs obtained during this initial phase then typically undergo a rapid expansion protocol ("REP"). The REP process increases the final number of TILs to the order of 10⁹ to 10¹¹ cells.

Adoptive cell therapy utilizing TILs cultured ex vivo by the Rapid Expansion Protocol (REP) has produced successful adoptive cell therapy following host immunosuppression in patients with cancer. In some instances, however, the survival and anti-tumor activity of the transferred TILs can decrease following transfer to the patient. Administration of supporting immunostimulatory agents (e.g., cytokines) has been explored to enhance T cell therapies. Such immunostimulatory agents, however, require high systemic doses that lead to undesirable toxicity. In addition, as part of Lifileucel^{®} treatment, two measures are taken to enhance the infused TILs ability to attack the cancer and their overall potency. First, in the days prior to receiving Lifileucel^{®}, patients undergo a few rounds of high-dose lymphodepleting chemotherapy. And second, shortly after the Lifileucel^{®} infusion, they are given several doses of IL-2. Current clinical experience of TIL therapy emphasis on lymphodepletion regimen and the administration of high-dose IL-2 and its related toxicity to amplify the therapeutic potency of TILs.

Also, Rosenberg et al describes a simplified method for initial TIL culture and a rapid expansion protocol in gas-permeable flasks. TILs are initially cultured from tumor digests and fragments in 40mL capacity flasks with a 10cm² gas-permeable silicone bottom, G-Rex10. A TIL rapid expansion protocol (REP) was developed using 500mL capacity flasks with a 100 cm² gas-permeable silicone bottom, G-Rex100. TIL growth was successfully initiated in G-Rex10 flasks from tumor digests from 13 of 14 patients and from tumor fragments in all 11 tumor samples tested. TILs could then be expanded to 8-10*10⁹ cells in a 2-step REP that began by seeding 5*10⁶ TIL into a G-Rex100 flask. TILs were cultured with irradiated (50 Gy) allogeneic PBMC as "feeder" cells at a ratio of 1 to 100 and the cells were cultured in a 1 to 1 mixture of CM and AIM-V medium (50/50 medium), supplemented with 3000 IU/mL of IL-2 and 30 ng/mL of anti-CD3. On day 7, cells from 2 T-175 flasks were combined in a 3L bag and 300mL of AIM-V with 5% human AB serum and 3000 IU/mL of IL-2 was added to the 300mL of TIL suspension. To obtain the 30-60*10⁹ cells used for patient treatment, 6 G-Rex100 flasks were seeded with 5*10⁶ cells and expanded into 18 G-Rex100 flasks (Jin et al. "Simplified Method of the Growth of Human Tumor infiltrating Lymphocytes in Gas-permeable Flasks to Numbers Needed for Patient Treatment. J Immunther 2012; 35:283-292. DOI: 10.1097/CJI.0b013e31824e801f).

Despite the conventional and classical TIL expansion served patients with cancer, there remains a need for improved TIL expansion and therapy for the treatment of cancers.

### Summary of the invention

The present invention provides an in-vitro or ex-vivo method of expanding lymphocytes comprising providing a population of lymphocytes, wherein the lymphocytes are from a biological sample; and culturing the lymphocytes in a growth medium; and expanding the lymphocytes in an expansion medium comprising nutrients and differentiation factors and/or cytokines, wherein the differentiation factors and/or cytokines comprise or consist of interleukin-2, interleukin-7, interleukin-15, and interleukin-21; and obtaining expanded lymphocytes. The obtained expanded lymphocytes are preferably tumor infiltrating lymphocytes (TILs). The invention further provides a population of expanded tumor infiltrating lymphocytes for use in a method for treating cancer, whereas the TILs are obtained by the method according to the invention.

### Figures

### Description of the Figures

Figure 1 depicts a flow-chart of the method according to the invention.
Figure 2 depicts the results for viability, purify, activity and potency of Cbl-b modified TILs.

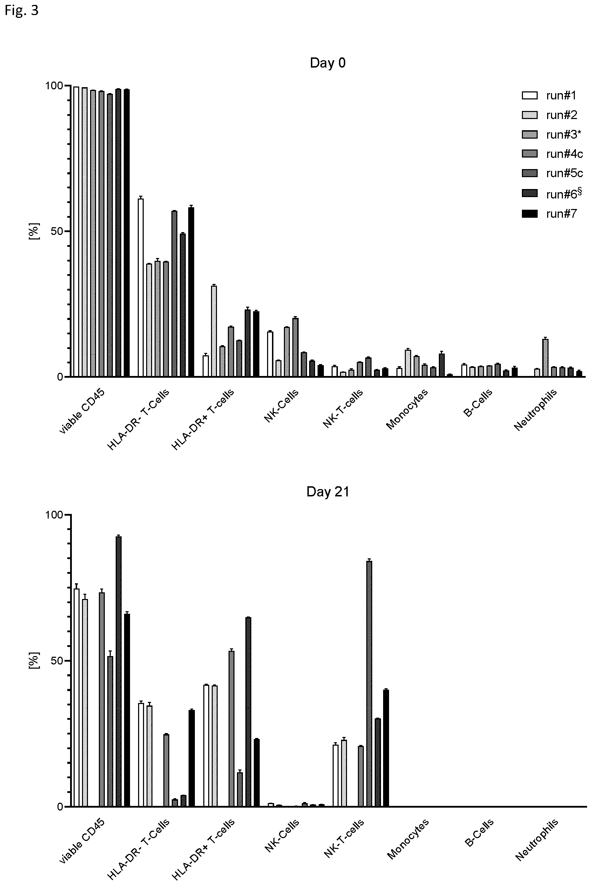

Figure 3 shows the immune composition of expanded cells using the inventive xpsTIL expansion protocol.

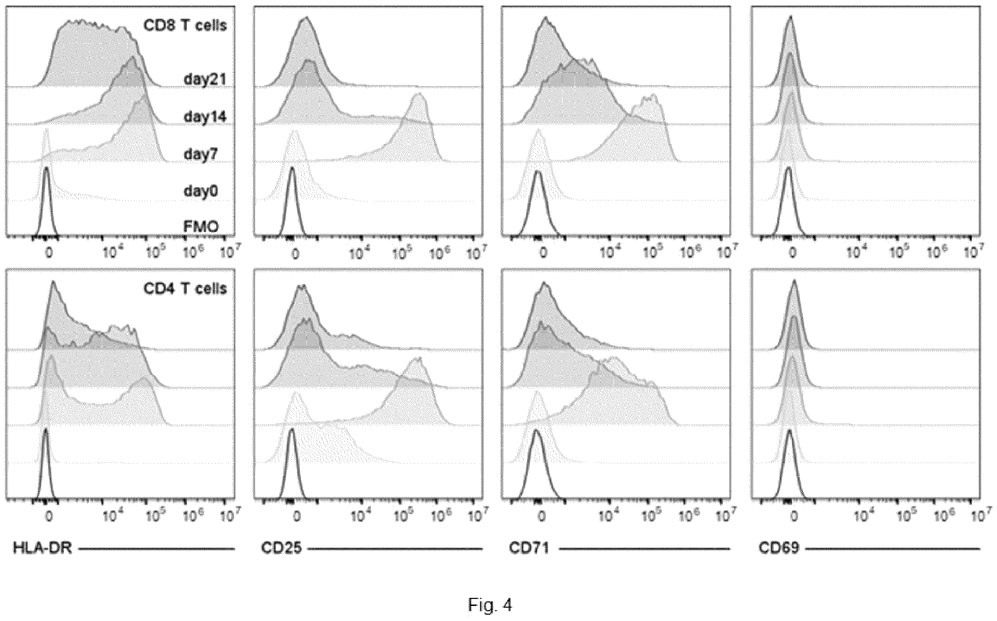

Figure 4 shows that T cells express markers of activation during xpsTIL expansion (run#1).

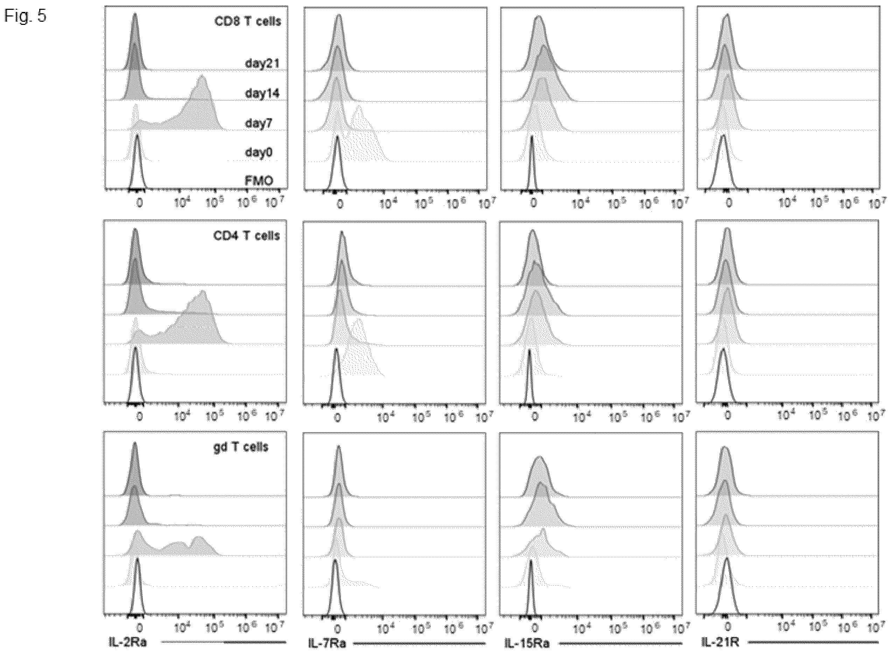

Figure 5 shows that T cells express receptors for cytokines IL-2, IL-7, IL-15 and IL-21 during xpsTIL expansion (run#1).

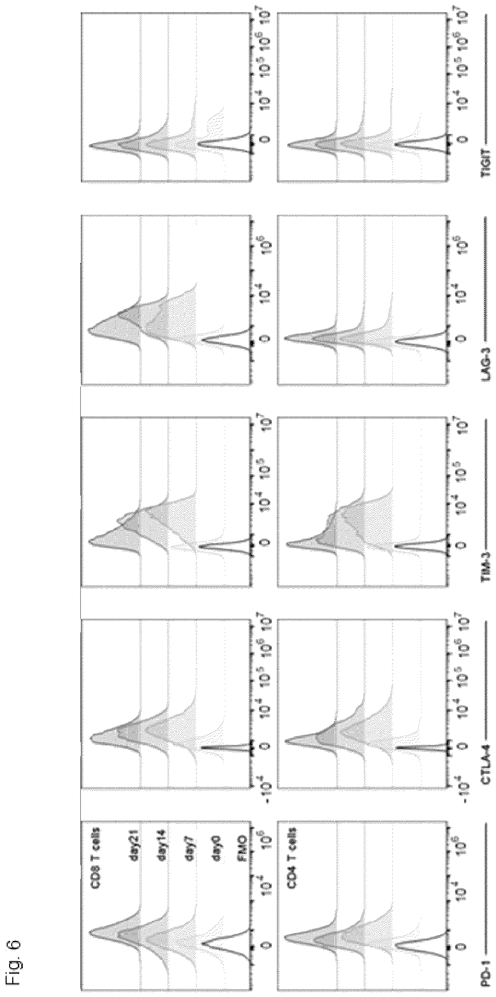

Figure 6 shows that T cells express immune checkpoints during xpsTIL expansion (run#1).
Figure 7 shows expression profiles of memory T-cell markers (CD45RO, CD45RA, CD62L, CD27, FAS) and an effector T-cell marker (CCR7) of lymphocytes during lymphocyte expansion.
Figure 8 shows that cytotoxic potency of expanded and Cbl-b silenced glioblastoma patient-derived T cells and TILs against M21 melanoma.
Figure 9 shows that cytotoxicity of expanded T cells towards human M21 melanoma (E:T = 1:1) is enhanced upon Cbl-b silencing.
Figure 10 shows that Cbl-b deficient mice control s.c. syngeneic GL261 GBM tumors.
Figure 11 shows that Cbl-b deficient mice control s.c. syngeneic SB28 GBM tumors.
Figure 12 shows that Cbl-b^{-/-} mice control orthotopic GL261 GBM tumors.
Figure 13 shows that Cbl-b^{-/-} mice control orthotopic SB28 GBM tumors.
Figure 14 shows that Cbl-b^{-/-} TILs expanded from s.c. GBM tumors show increased cytotoxicity compared to T cells or WT TILs.
Figure 15 shows that Cbl-b^{-/-} TILs expanded from i.c. SB28 tumors show increased cytotoxicity compared to WT TILs.
Figure 16A-C show that transfer of expanded Cbl-b^{-/-}(ΔCbl-b) TILs reduces orthotopic GL261 GBM growth and prolongs survival.
Figure 17 shows that transfer of expanded Cbl-b^{-/-}(ΔCbl-b) TILs reduces orthotopic SB28 GBM growth.
Figure 18 shows that with the method of the invention CD45 leukocytes can be expanded from glioblastoma tumor tissue.

### Description

### Detailed description of the invention

An in-vitro or ex-vivo method of expanding lymphocytes comprising providing a population of lymphocytes. The lymphocytes are from a biological sample such as from a patient. The patient may be in need of treatment with expanded lymphocytes of any embodiment of the invention. The inventive method comprises culturing the lymphocytes in a growth medium; and at least one subsequent step of expanding the lymphocytes in an expansion medium comprising nutrients and differentiation factors and/or cytokines, wherein the differentiations factors and/or cytokines comprise or consist of interleukin-2 (IL-2), interleukin-7 (IL-7), interleukin-15 (IL-15), and interleukin-21 (IL-21) or a variant thereof. Then expanded lymphocytes can be obtained. The method of the invention enables the production of TILs at numbers required for patient treatment at cell processing laboratories. The expanded lymphocytes may comprise phenotypic shifted lymphocytes. Phenotypic shifted lymphocytes have an altered phenotypic and/or functional state than before the application of the inventive method, such as an altered activation and/or immunoreactivity. The expansion culture is expected to shift the phenotype of TILs towards memory or even stem cell-like memory. This is further intended to prolong the in vivo persistence after adoptive cell transfer. In particular, the inventive treatment with IL-2, IL-7, IL-15, and IL-21 usually leads to a prolonged high reactivity against antigens. Administration of supporting immunostimulatory agents, e.g., IL-2, has been explored to enhance T cell therapies. Such immunostimulatory agents, however, require high systemic doses that lead to undesirable toxicity. Survival of transferred TILs generated by the method according to the invention is ensured even without additional administration of IL-2 to the patient and without prior lymphodepletion.

The immunomodulatory agents associated with the culturing and expansion method of TILs provide an immunostimulatory effect that can advantageously enhance TIL survival, proliferation and/or anti-tumor activity in a patient recipient. As such, the compositions and methods disclosed herein provide effective cancer therapies.

The type of lymphocytes directed against a specific antigen is usually not altered in the inventive method. There may be a selection step to select lymphocytes that are reactive against a particular antigen. In other embodiments there may not be such a selection step, such as when the obtained lymphocytes can be expected to be reactive against an antigen that is of interest, such as when the reactivity against the antigen is of therapeutic interest.

The lymphocytes may comprise lymphocytes with a chimeric antigen receptor (CAR), such as CAR T cells. CAR T cells may target an antigen via the CAR or T cell receptor.

The lymphocytes may be in a population of peripheral blood mononuclear cells (PBMCs). PBMCs may comprise lymphocytes selected from T cells, B cells, NK cells. PBMCs may further comprise non-lymphocytes, such as monocytes, granulocytes, or dendritic cells. Lymphocytes are usually the majority in biological PBMC samples. The biological sample may be a sample comprising leukocytes such as a body fluid, like blood, interstitial fluid, cerebrospinal fluid (CSF) or liquor. Preferably the lymphocytes are T-cells and/or B cells.

The biological sample comprising the lymphocytes may be from a patient suffering from a disease. Fresh tumor tissue is obtained through surgery and is processed in a reasonable amount of time. For initial growth of TILs, tumor tissue can be cut into fragments or made into a single-cell suspension through enzyme digestion, physical disaggregation or fine needle aspiration. The tumor fragments or single-cell suspension are then cultured in a growth medium. The disease may be linked to an antigen, such as an antigen from a pathogen or a tumor-associated antigen. The lymphocytes preferably comprise immune cells that are directed or reactive against such a disease-linked antigen.

In the inventive method, the step of culturing the lymphocytes in a growth medium serves to activate the lymphocytes. In a preferred embodiment of the invention the lymphocytes are not isolated but grow specifically from the heterogeneous cell population. The tumor location may affect the production of TILs, e.g they may be contaminated by microbes or may contain large numbers of ineffective T cells when originating from lymph nodes. Usually, the lymphocytes are delivered to a facility performing the inventive method after a transportation step from hospital, where the biological sample has been isolated from a patient. After a transportation step, the lymphocytes may have entered a resting stage. The first growth step serves to activate the lymphocytes, making them more receptive towards the further treatment of the invention that follows. In this step the lymphocytes, especially preferred the T cells, exit a resting phase and are conducive to proliferation survival and persistence of TILs in vivo. These unselected TILs have no preselection step for tumor reactivity, e.g. based on IFN-γ. This step may be optionally omitted, such as if the lymphocytes are already active.

In preferred embodiments the non-expanded lymphocytes are treated with a CD3 activator and/or a CD28 activator by incubation with an anti-CD3 agonist and or an anti-CD28 agonist, preferably with an activating anti-CD3 antibody and/or an activating anti-CD28 antibody. Such an activation can be done in a medium containing TransACT and/or activating anti-CD3/anti-CD28 antibodies. To an example growth medium can be added T Cell TransAct^{®} (see example 2.5). The growth medium shall activate the non-expanded lymphocytes and initiate a growth stage in the lymphocytes. Preferably, the growth medium activates T cells via CD3 and/or CD28 stimulation. The addition of TransAct^{®} facilitates proliferation of TILs by TCR activation. The activation after the expansion further increases activity, including a long-lasting activity, against a target, such as an antigen. Such as CD3 and/or CD28 activation can also be performed during expanding the lymphocytes, especially in the later days of this step, such as after 15 days.

Preferably the step of culturing the lymphocytes in the growth medium is performed without the addition of immunomodulatory agents including cytokines.

The step of culturing the non-expanded lymphocytes in a growth medium may be performed for 8 to 60 hours, preferably for 10 to 48 hours, more preferred for 16 to 36 hours, especially preferred for 20 to 28 hours, such as about 24 hours.

Preferably 0.05×10⁶ to 50×10⁶ lymphocytes, preferably 0.5×10⁶ to 5×10⁶ lymphocytes, per mL are transferred into a culturing vessel. Preferably the lymphocytes comprise 0.01×10³ to 10×10⁶ CD3⁺ immune cells, per mL. The CD3⁺ immune cells are preferably T cells, including cytotoxic T cell (CD8⁺ T cells) and/or T helper cells (CD4⁺ T cells). This step may be performed in a growth medium comprising nutrients, a buffer, and isotonic salts, and optional stabilizers.

In the step of expanding the lymphocytes in a medium comprising nutrients and differentiation factors and/or cytokines, wherein the differentiations factors and/or cytokines comprise or consist of IL-2, IL-7, IL-15, and IL-21 (the medium also being referred to as "expansion medium"), the lymphocytes are shifted towards a memory phenotype which has the benefit of long-lasting activity. This means that the activated lymphocytes remain active for a longer period of time in the patient (after the cells have been transferred to the patient for a treatment). The combination of a treatment of the lymphocytes with IL-2, IL-7, IL-15, and IL-21 has particularly beneficial effects towards this long-lasting effect. Preferably no other cytokines are used in this step that would mitigate this effect. Other components for growth or maintenance of lymphocytes, such as nutrients, a buffer, and salts, such as isotonic salts, optional stabilizers, like a polymer, albumin or preserving agent, may of course be added. Albumin is preferably human serum albumin. The expansion of lymphocytes in medium results in the dilution of TransAct^{®} thereby ceasing TCR stimulation.

The step of expanding the lymphocytes in an expansion medium comprising nutrients and differentiation factors and/or cytokines may be performed for at least 7 days, preferably at least 10 days; and/or the step may be performed for 7 to 40 days, preferably for 11 days to 30 days, preferably for 16 days to 25 days. The expansion is continued until a final cell population of at least 1×10⁷ cells is achieved.

Common protocols of adoptive cell therapy usually require expanding cells ex vivo for several weeks and/or involves the use of multiple culture phases wherein the cells are typically frozen between phases to reach sufficient cell numbers (approximately 10⁹ cells). The method of the invention avoids the freeze thawing where a large fraction of the cells may be lost. Additionally prolonged culturing that can cause T cells to become terminal effector cells that may die shortly after infusion to the patient before reaching a target cell, tissue and/or organ can also be circumvented. Advantageously the population of TILs produced by the method of the invention is not terminally differentiated and comprises a low fraction of terminal effectors.

A further advantage of the method of the invention is that a possible other immune status of the patient after a long expansion period of the cells before infusion of cells into the patient can be considered.

Preferably 0.05×10⁶ to 50×10⁶ lymphocytes, preferably 0.5×10⁶ to 5×10⁶ lymphocytes, per mL are expanded in the expansion medium of a culture vessel. Preferably the lymphocytes comprise 0.1×10³ to 10×10⁶ CD3⁺ immune cells, per mL. The CD3⁺ immune cells are preferably T cells, including cytotoxic T cell (CD8⁺ T cells) and/or T helper cells (CD4⁺ T cells). Preferably IL-2 is present in the medium in a concentration of 2000 IU/mL to 5000 IU/mL, preferably 2500 IU/mL to 3500 IU/mL, especially preferred about 3000 IU/mL. Preferably IL-7 is present in the medium in a concentration of 2 ng/mL to 10 ng/mL, preferably 3.5 ng/mL to 7.5 ng/mL, especially preferred about 5 ng/mL. Preferably IL-15 is present in the medium in a concentration of 2 ng/mL to 10 ng/mL, preferably 3.5 ng/mL to 7.5 ng/mL, especially preferred about 5 ng/mL. Preferably IL-21 is present in the medium in a concentration of 2 ng/ml to 10 ng/mL, preferably 3.5 ng/mL to 7.5 ng/mL, especially preferred about 5 ng/mL. In a preferred embodiment of the invention the expansion medium comprises a cytokine cocktail comprising or consisting of 3000 IU/mL IL-2, 5 ng/mL IL-7, 5 ng/mL I_-15, and 5 ng/mL IL-21.

Preferably the differentiation factors and/or cytokines do not include interleukin-4 (IL-4) and/or do not include interleukin-17 (IL-17). IL-4 and IL-17 would tend to induce a suppressive phenotype in T cells, which is not desired. The invention intends the opposite, i.e. an activated phenotype. Of course, negligible amounts of IL-4 and IL-17 can be allowed when the activation of the lymphocytes is not hindered.

Especially preferred, the lymphocytes comprise CD4⁺ T-cells and/or CD8⁺ T-cells. Such T-cells provide an excellent anti-tumor activity and are very useful in a therapy targeting a disease-associated antigen. These cells can also be efficiently activated using the inventive method.

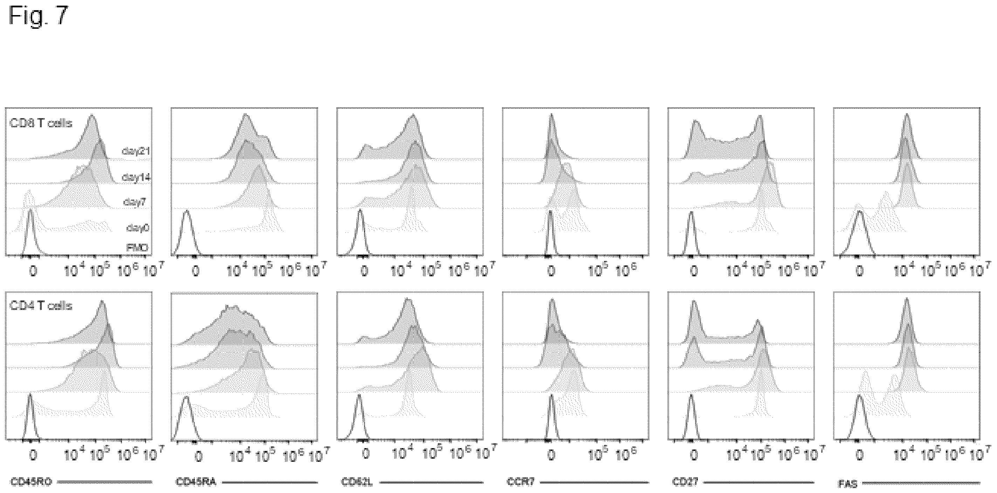

Preferably the step of expanding the lymphocytes (to yield the required number of expanded lymphocytes) is performed for a duration until at least 60% of the lymphocytes, preferably at least 60% of the T-cells when the lymphocytes comprise T-cells, express CD45RO, CD45RA, CD62L and/or FAS. CD45RO, CD45RA, CD62L and FAS are expression markers of memory cells (see Figure 7) and show that the inventive method has efficiently phenotypic shifted the lymphocytes. CD27 may also be expressed by the lymphocytes. CD27 may be present on CD8⁺ T-cells, preferably in at least 30% of the CD8⁺ T-cells.

Preferably in the expanded lymphocytes at least 50% of the T-cells that express CD45RO, CD45RA, CD62L and/or FAS do not express CCR7 or have a low expression of CCR7. A low expression of CCR7 may be a negligible expression. CCR7 is a marker of effector T-cells. It contrasts memory T cells that usually do not express this marker. Preferably CCR7 is expressed by less than 5% of the T cells when the step of expanding the lymphocytes is finished. CCR7 may be expressed in some cells in early stages of activation (e.g. day 0-7) but later should end in phenotypic shifted lymphocytes, especially in T cells.

Preferably the expanded lymphocytes comprise memory T cells and/or do not comprise effector T cells or do comprise effector T cells at an amount of less than 20% of the expanded lymphocytes. CD27 and CD28 are expressed in memory T cells but not in terminal effector T cells and may also be used as markers for the determination of type of expanded and phenotypic shifted tumor infiltrated lymphocytes.

The percentage of markers in the population of expanded tumor infiltrating cells is preferably determined by flow cytometry using antibodies directed against the corresponding markers.

Alternatively, or in addition to the markers CD45RO, CD45RA, CD62L and/or FAS, the population of lymphocytes according to the invention may be characterized based on the secretion profile of the CD3+ T cells comprised in the population of lymphocytes. It is known in the art that terminal effector T cells secrete different proteins than less differentiated T cells. Accordingly, phenotypic shift of the cells in a population of cells may be determined based on the proteins that are secreted by the cells in the population, e.g. TNF-α, Granzyme B and Perforin (Wang et al. Stem cell-like memory T cells: The generation and application. J Leukoc Biol. 2021; 110:1209-1223. https://doi.org/10.1002/JLB.5MR0321-145R).

In a particular embodiment, the invention also relates to a population of lymphocytes for autologous cell transfer in humans, the population of lymphocytes comprising at least tumor infiltrating T cells, wherein at least 80% of said T cell portion are viable and at least 90% are at least double positive, preferably triple positive, for CD45RO, CD45RA, CD62L and/or FAS, respectively. The population of tumor infiltrating cells generated according to the method of the invention exhibits low percentages of terminal effector cells, that are known to have limited capacity of replication but high cell killing activity.

The term "expanded lymphocytes" refers to the lymphocytes after the step of expanding the lymphocytes. In this step the lymphocytes are expanded until the cells have this cell composition or marker profile or the cytokines are adjusted until the cells have this cell composition or marker profile.

In a preferred embodiment of the invention the TILs undergo a phenotypic shift that causes expression of one or more immune checkpoint genes to be silenced or reduced in at least a portion of the therapeutic population of TILs. In some embodiments of the invention said one or more immune checkpoint genes is/are selected from the group comprising Cbl-b, PD1, PD-L1, PD-L2, GASP, CTLA4, FoxP3, LAG- 3, TIM-3, TIGIT, A2AR, KIR, BTLA, VISTA, B7-H3, B7-H4, SHIP, SHP-1, SHP-2, IL-1R8, NKG2A,CD96, CD112R, IDO, IRG-1, STAT-3, JAKs, Arg-1, Nos-2, Gish, TGFβ, PKA, TNFRSF, Cish, PPP2CA, PPP2CB, PTPN6, PTPN22, PDCD1, BTLA, CD160, TIGIT, TET2, CD96, CRTAM, LAIR1, SIGLEC7, SIGLEC9, CD244, TNFRSF10B, TNFRSF10A, CASP8, CASP10, CASP3, CASP6, CASP7, FADD, FAS, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3, TOX, SOCS1, ANKRD11,NR2F6, TMEM222, SOCS1, CDKN1, CD5, UBASH3A, RASA2, CD5, TNFAIP3, TCEB2, TET2 and BCOR, preferably encoding for the human Cbl-b, or a combination thereof. The target nucleic acid sequence can be the sequence of a further immunosuppressive regulator, chemokine receptor and/or cytokine or a combination thereof. Any of these immune checkpoint genes, immunosuppressive regulators, chemokine receptors can be targeted by the transfection nucleic acid, especially the inhibitory nucleic acid.

The invention may further include CAR-T cells engineered by methods known from the state of the art, preferably mRNA transfection, for example expressing chimeric antigen receptors (CARs) against EGFR, NY-ESO, CEA, GD2 for transient protein expression.

The downmodulation of immune inhibitory molecules (immune suppressive molecules) may be produced using one or more methods selected from an RNA interference method (e.g., siRNA), a CRISPR method, a TALEN method, a zinc finger method, a Cas-CLOVER method, and/or a combination thereof.

In an alternative embodiment of the invention, it is possible to induce immune modulatory molecules, e.g. with mRNA molecules or the methods or combinations thereof mentioned above.

In a further embodiment of the invention the modified TILs transiently express the immunomodulatory composition on the cell surface.

In a preferred embodiment the expanded TILs are modified by transfecting the TILs with a nucleic acid against or to silence an immune checkpoint. The TILs are transfected with the siRNA by electroporation after the expansion. Electroporation can be performed using methods and devices known from the state of the art (e.g. MaxCyte, Thermo Fisher, Miltenyi Biotec, etc.).

In especially preferred embodiments, the inventive method further comprises the step of inhibiting Cbl-b in the lymphocytes. Silencing of this checkpoint not only boosts immune cell function, but also prolongs the time until exhaustion and renders the modified cells partially resistant against suppressive cues in the tumor microenvironment which is prominent in glioblastoma patients. This step can be performed as described in WO 2009/073905 A2 or in WO 2023/237764 A1. Preferably inhibition of Cbl-b is done during or after the step of expanding the lymphocytes, preferably after expanding the lymphocytes for at least 7 days. Casitas B lineage lymphoma proto-oncogene b (Cbl-b) is an E3 ubiquitin ligase involved in inhibition of signaling by protein-tyrosine kinases. In T lymphocytes, Cbl-b is induced after TCR ligation and PD-1 and CTLA-4 ligation and negatively regulates T cell activation (via PKC, PLC, Vav and Nedd4) and CD28 signaling. Loss or silencing of Cbl-b uncouples TCR signaling from the requirement of CD28 co-stimulation for T lymphocyte proliferation and effective IL-2 production. Furthermore Cbl-b deficient T cells are non-responsive to suppressive mechanisms by regulatory immune cells and their released factors. Therefore Cbl-b represents an important intracellular immune checkpoint in T lymphocytes and other leukocytes and knock-out or transient knock-down renders T lymphocytes hyper-active and ignorant to suppressive cues present in the tumor micro-environment. Preferably inhibiting Cbl-b is a transient inhibition of Cbl-b, such as by knock-down, especially by silencing, especially preferred by using a siRNA. Anti-Cbl-B siRNA is e.g. described in WO 2009/073905 A2 or WO 2023/237764 A1. Preferably the step of inhibiting Cbl-b is in a closed container system as described in WO 2023/237764 A1.

Preferably the population of lymphocytes is a purified population of lymphocytes. Preferably the lymphocytes are in a sample with cells and at least 80% of the cells in the sample are lymphocytes, preferably at least 80% of the cells in the sample are T-cells. Such populations can be obtained from suitable biological fluids from a patient, with optional enrichment of lymphocytes or T-cells if needed. The leukocytes may be obtained by apheresis, especially leukapheresis.

Preferably the lymphocytes are mammalian lymphocytes, preferably human lymphocytes. Preferably the patient is a mammal, preferably a human. When used in therapy, the lymphocytes are preferably autologous such as in an autologous immune enhancement therapy.

The elements as described above can be combined. E.g. in a preferred embodiment, the lymphocytes are CD3+ T-cells from a tumor or tumor-associated liquid of a mammal; and culturing the lymphocytes in a growth medium comprises culturing the T-cells in a medium comprising or consisting of nutrients, a proliferation agent, a buffer, and isotonic salts, and optional stabilizers; and expanding the lymphocytes comprises expanding the T-cells in an expansion medium comprising or consisting of nutrients, a buffer, and isotonic salts, optional stabilizers, and differentiation factors and/or cytokines consisting of interleukin-2, interleukin-7, interleukin-15, and interleukin-21. Any of these steps are preferably further characterized as described above.

Provided herein is a method of culturing and expanding tumor infiltrating lymphocytes (TILs) for production of a therapeutic population of TILs, the method comprising the steps of:
a) adding the tumor fragments or single cell suspension into the growth medium without cytokines;
b) culturing a population of TILs from a fluid or a tumor sample resected/or biopsied from a cancer in a subject by processing the tumor sample obtained from the tumor into multiple tumor fragments or a single cell suspension by performing an activation by culturing the population of TILs in a growth medium comprising a T cell activator to produce a population of TILs, wherein the culturing is performed preferably in a closed container for about 24 hours to obtain the further population of TILs;
c) performing a pre-expansion by culturing the first population of TILs in a cell culture expansion medium comprising IL-2, IL-7, IL-15 and IL-21 to produce a second population of TILs, wherein the pre-expansion is performed in a multiple well plate, preferably in a closed container, and is performed for about 1 to 7 days to obtain the second population of TILs;
d) performing an expansion by supplementing the cell culture expansion medium of the second population of TILs with cell culture expansion medium with additional IL-2, IL-2, IL-7, IL-15 and IL-21, to produce a third population of TILs, wherein the expansion is performed for about 7 to 14 days to obtain the third population of TILs, wherein the expansion is performed in a closed container
e) harvesting and transferring the third population of TILs obtained from step (d) to a sample collection bag, wherein the transition from step (d) to step (e) occurs without opening the system;
f) modifying the third population of TILs after the transfer to the sample collection bag in step (e) to generate modified TILs.

In a further aspect, provided herein is a method of treating a cancer in a patient or subject in need thereof comprising administering a population of modified tumor infiltrating lymphocytes (TILs) produced by the method described above.

Preferably the lymphocytes are lymphocytes from a tumor and/or from a fluid of a tumor; and/or wherein the lymphocytes are tumor infiltrating lymphocytes (TILs). In an alternative embodiment of the invention the lymphocytes may be pre-isolated from the tumor or fluid of a tumor. Preferably the tumor is a glioblastoma. The lymphocytes can comprise or consist of tumor infiltrating lymphocytes (TILs). TILs may be obtained from preparations of primary tumors and/or metastasis and/or spinal or cerebrospinal fluid.

The invention also provides expanded lymphocytes, especially expanded and phenotypically shifted tumor infiltration lymphocytes, obtained or obtainable by any one of the inventive methods. At least 80%, preferably 90%, most preferably 95% of the lymphocytes are viable tumor infiltration cells after the expansion of cells.

The expanded lymphocytes can be used as a medicament, especially in the treatment of a disease. Such a treatment may be an adoptive cell therapy. The provided population of lymphocytes may be from a patient and/or the biological sample may be from a patient. The patient is preferably a mammal, especially preferred a human. Within this aspect, the expanded lymphocytes may be for use in a therapy, especially in a treatment of the disease. The expanded lymphocytes may be used for the manufacture of a medicament for the treatment of a disease. The invention also provides the population of lymphocytes as disclosed herein within a pharmaceutically acceptable carrier in the form of a pharmaceutical composition. The medicament and pharmaceutical compositions as disclosed herein are, in particular, for use in adoptive cell therapies.

A disease to be treated may be cancer. The lymphocytes may be from a biological liquid and/or from a resected tumor of a patient. The lymphocytes may be tumor infiltrating lymphocytes and/or the lymphocytes may be PBMCs (peripheral blood mononuclear cells). The lymphocytes may be from a tumor-associated liquid. The lymphocytes can be obtained by apheresis from a patient, preferably by a leukapheresis procedure. Tumor infiltrating lymphocytes can be obtained from processed tumor fragments or tumor associated liquids. The leukapheresis product from a respective patient can be received in a container, preferably in an out-put bag from leukapheresis. The leukapheresis sample contains preferably a target cell number of approximately 1×10⁶ to 1×10¹⁰ PBMCs/kg, preferably 1.2×10⁸ PBMCs/kg in approximately 100 to 500 mL, preferably 200 mL in the biological sample. In respect of leukapheresis a high volume of PBMCs is preferable. PBMCs/kg refers to the number of PBMCs per kg body weight of the patient (subject).

The cancer is preferably glioblastoma. Glioblastoma is the most common and lethal primary brain tumor in adults. Only 5% of patients survive more than 5 years. Current standard of care includes maximal safe resection followed by fractionated radiotherapy combined with daily chemotherapy with temozolomide (TMZ). No significant improvements have been made in the past 20 years apart from adding chemotherapy to radiotherapy as the standard of care. Glioblastoma contains only a few coding mutations and shows high intratumor heterogeneity hampering attempts to target tumor-intrinsic driver mutations. Apart from that, the protection by the blood-brain-barrier (BBB), unique features of the brain rendering it immunologically distinct, and the highly suppressive tumor-microenvironment (TME) of brain tumors pose challenges for the development of efficacious immunotherapies. Yet the inventive expanded lymphocytes are highly suited for the treatment of glioblastoma. The biological sample may be cerebrospinal fluid (CSF) or liquor harvested from a glioma or glioblastoma patient.

The modified TILs may be used in a pharmaceutical composition comprising the modified TILs of any one of claims. Using the pharmaceutical composition about 1×10⁶ to 5×10⁸, preferably 5×10⁶ to 5×10⁷ expanded TILs per mL in a single dose application may be administered intracranially via an implanted Ommaya Reservoir. Repeated sampling of source material for the manufacturing assembly procedure and repeated intraventricular administrations via implanted Ommaya Reservoir is possible with TILs produced according to the method of the invention. Furthermore, the locoregional application of expanded TILs via an Ommaya reservoir bypasses the need for efficient trafficking to the tumor site, which poses a significant obstacle in glioblastoma since the brain is an immune privileged organ that additionally produces a highly suppressive tumor microenvironment in this hard-to-treat indication.

TILs expanded with the method according to the invention may be combined with standard of care and potential combination therapies with other anti-cancer modalities, e.g. dendritic cells, anti-MDSC drugs, immune checkpoint inhibitors, immune cells expressing chimeric antigen receptors. As used herein, combination with the population of expanded tumor infiltration lymphocytes, medicaments and/or pharmaceutical compositions of the invention does not necessarily indicate that lymphocyte therapy and one or more additional medicaments need to be administered together, e.g. in the same infusion. Combination includes concomitant and sequential administration in any order. Combination also includes dosing schemes wherein one or more agents are administered multiple times over the time frame, e.g. of days, weeks, or months, and the other agent or agents are administered only once or in according to a different dosing scheme. Combination includes any scheme wherein the agents are purposefully administered so that the therapeutic effects overlap at least to some extent.

Any of the growth media or the obtained lymphocyte may be admixed with a pharmaceutically acceptable carrier excipient and/or diluent. The carrier must, of course, be acceptable in the sense of being compatible with any other ingredients in the formulation and must not be deleterious to the lymphocytes. Examples of suitable pharmaceutical carriers are well known in the art and include phosphate buffered saline solutions, water, emulsions, such as oil/water emulsions, various types of wetting agents, sterile solutions etc. The carrier may be a solution that is isotonic with blood, especially human blood. Compositions comprising such carriers can be formulated by well-known conventional methods. Preferably any of the media or the preparation of the obtained lymphocytes may comprise salt, such as NaCl, especially 0.9% NaCl (wt.-%), a stabilizing agent like albumin, and/or a carbohydrate source.

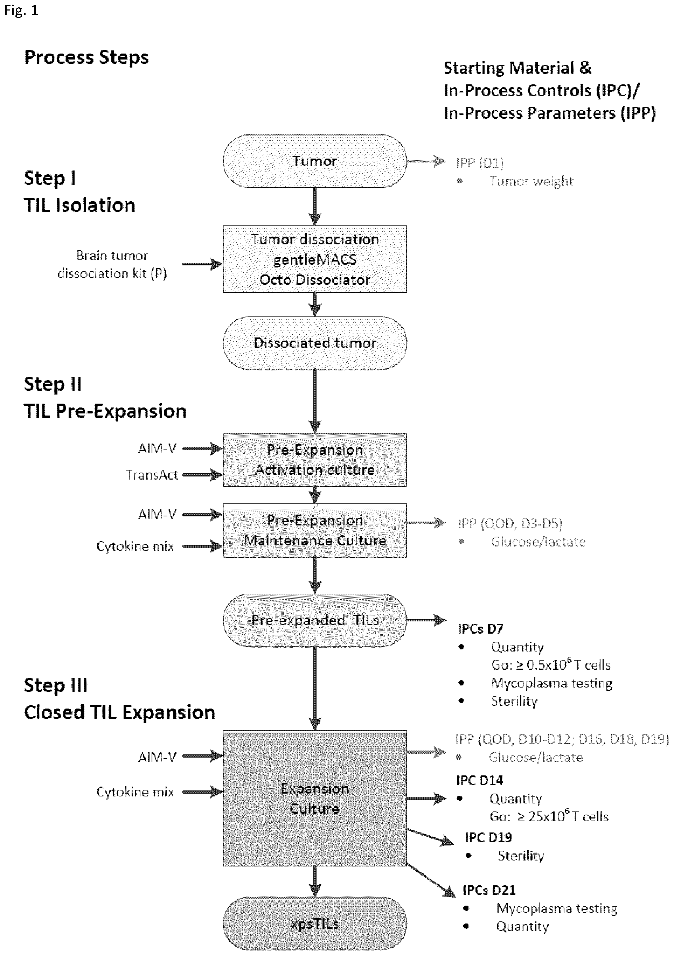

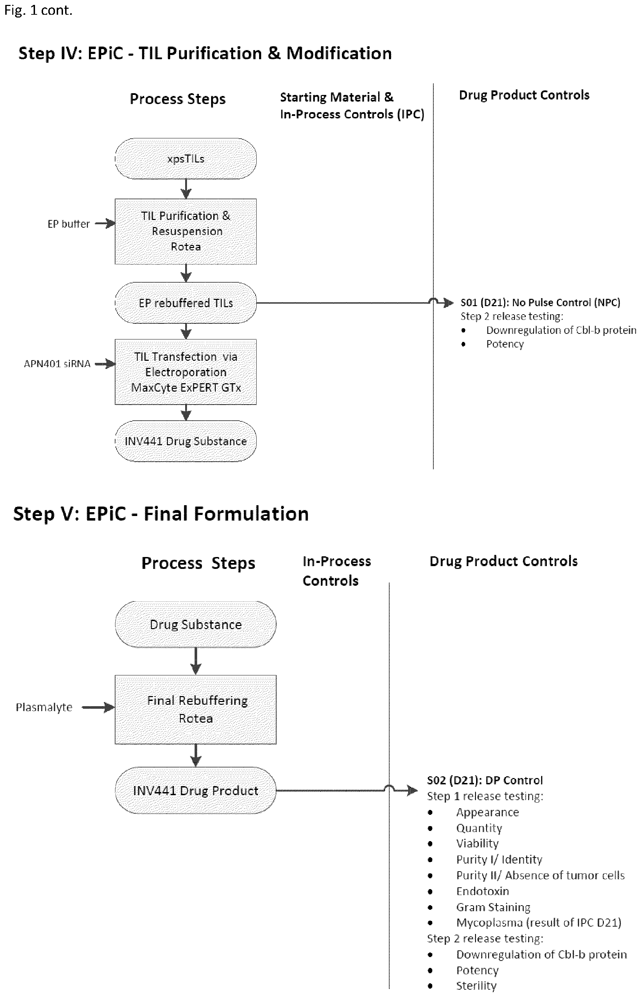

Figure 1 depicts an overview in the form of a flow chart of the method according to the invention.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range and "comprising" relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further illustrated by the following figures and examples, without being limited to these embodiments of the invention.

The aim of these experiments was to develop and describe a cell expansion process in a closed system that allows culture of cells directly from cerebrospinal fluid (CSF)/liquor material harvested from Glioma or Glioblastoma patients via an Ommaya Reservoir or from digested Glioblastoma tumor tissue. In addition to cell expansion the lymphocytes, especially the T cells, are shifted towards a memory phenotype which has the benefit of long-lasting tumor control in patients treated with expanded TILs according to the invention.

### Examples

### Example 1: Cbl-b modified and expanded and phenotypic shifted (xps) TILs

The population of cells according to the invention describes a suspension of autologous, viable, ex vivo expanded tumor infiltrating lymphocytes (TILs) from an individual patient that have been transfected with an siRNA in order to reduce Cbl-b expression. Expanded and transfected autologous TILs are infused back into the neurooncological patient via a pre-implanted Ommaya reservoir. Cbl-b functions as a negative regulator of innate and adaptive immunity, thereby facilitating tumor-induced immune evasion. Consequently, the inhibition of Cbl-b function following the silencing of the Cbl-b gene in immune cells may result in the reactivation of immune processes that facilitate the eradication of tumor cells. The administration of autologous, ex vivo expanded and Cbl-b silenced TILs represents a novel approach to enhancing anti-tumor immune responses, with the potential to improve cancer therapy.

### Preparation of tumor tissue

A study nurse may be tasked with the collection of the sample. Therefore, it is recommended to fill 4 sterile 25 mL tubes with 5mL sterile PBS in a class A laminar air flow hood. Each tube has to be weighted and weight (tara) has to be documented on the Batch Manufacturing record. Subsequently, the tubes are to be precooled in the cooling container. During the operation, the study nurse receives the tumor pieces and transfers them immediately to the 25 mL tubes. However, this should be done quickly and the tissue should be stored in the cooling container. For additional tissue a second or even a third tube should be used. Afterwards the tubes containing tissue are going to be weighted. The final tumor weight is determined by subtracting the weight of the tube containing tissue from the tara. The weight of all tissue-containing tubes, minus the tara, is then summed.

### Processing of human brain tumor tissue

The generation of a single-cell suspension containing tumor infiltrating lymphocytes (TIL) is achieved through the usage of the Brain tumor dissociation Kit Enzyme Mix (Miltenyi Biotec #130-095-942) and the gentleMACS^{™} Dissociator with Heaters. In summary, the operator receives the tumor tissue with the final calculated weight. The operator adds preheated Buffer X to the C tube in accordance with the calculated weight. In the event that the quantity of tumor tissue received exceeds 1,600 mg, it is necessary to use two C tubes. Subsequently, the tumor tissue is transferred to a sterile culture dish and cut into 2-4 mm pieces with a single use scalpel. The pieces are transferred to a GentleMACS C tube containing the appropriate amount of Buffer X. Subsequently Buffer Y, Enzyme N and A are added. The C tube is placed in a gentleMACS Dissociator and program 37C_BTDK_1 (~22min) is started. Following this, the sample is briefly centrifuged at 460 rcf, resuspended in AIM V complete medium and transferred through a 70 µm into a 50 mL tube.

### Expansion of human TILS

In this phase of the process, the single cell suspension is cultivated to obtain expanded Tumor Infiltrating Lymphocytes. TILS are cultivated with G-Rex devices. The process is controlled via glucose/ lactate measurements with e.g. the Hitado Super GL device on days 3, 5, 8, 10, 12, 16, 18. On Day 7, 14 and 21 consumed medium is collected, cells are resuspended in remaining medium and a small number of cells is taken for BD TruCount analysis using flow cytometry. The expansion process can be divided into two phases: the pre-expansion phase and the expansion phase.

### Pre-Expansion

In the event that the tumor tissue weighs less than 800 mg, the cells are plated in a G-Rex 24 well plate. In brief, digested single cell suspension is centrifuged at 460 rcf for 5 min at 4°C. The supernatant is discarded and the cells are resuspended in 2 mL AIM V Activation medium containing 20 µL TransACT. Following a 24 hour incubation, 6 mL Expansion Medium (AIM V complete containing 4000 IU/mL IL-2, 6.67 ng/mL IL-7, 6.67 ng/mL IL-15, 6.67 ng/mL IL-21) is added to the well.

In the event that the tumor weighs between 801- 1600 mg, the cells are transferred to a G-Rex 5M. Therefore, the digested single cell suspension is centrifuged at 460 rcf for 5 min at 4°C. The supernatant is discarded, cells are resuspended in 5 mL AIMV Activation medium containing 50 µL TransACT and cells are incubated for 24 hours. After 24 hours, 45 mL Expansion Medium (AIMV complete containing 3333.333 IU/mL IL-2, 5.55 ng/mL IL-7, 5.55 ng/mL IL-15, 5.55 ng/mL IL-21) is added to the G-Rex 5M.

In the event that the tumor weighs more than 1600 mg, the cells are transferred to a G-Rex 10M. In brief, the digested single cell suspension is centrifuged at 460 rcf for 5min at 4°C. The supernatant is discarded and cells are resuspended in 10 mL AIM V Activation medium containing 100 µL TransACT. Following a 24 hour incubation at 37°C and 5% CO₂, 90 mL Expansion Medium (AIM V complete containing 3333.333 IU/mL IL-2, 5.55 ng/mL IL-7, 5.55 ng/mL IL-15, 5.55 ng/mL IL-21) is added to G-Rex 10M.

The process is regulated by the measurement of glucose and lactate levels on days 3 and 5. In the event that the lactate concentration exceeds 12 mM/L, a medium change is required.

For tumor tissue <800mg, 6 mL of medium is carefully collected and 6 mL of Expansion Medium (AIM complete containing 3000 IU/mL IL-2, 5 ng/mL IL-7, 5 ng/mL IL-15, 5 ng/mL IL-21) is added. For tumor tissue >800mg consumed medium is carefully collected with a sterile pipette or the GatheRex device. Afterwards Expansion Medium (AIM complete containing 3000 IU/mL IL-2, 5 ng/mL IL-7, 5 ng/mL IL-15, 5 ng/mL IL-21) is added.

On day 7 consumed medium is collected from the G-Rex device and the cells are carefully resuspended in remaining volume. Additionally, samples for sterility testing and mycoplasma-testing and for further analysis are drawn.

*Day* 7 *Flow cytometry for In Process Controls and Release Testing* Cell count is conducted using BD-TruCount tubes. Therefore, a small number of cells (50 pL) is taken from the G-Rex System. 10 µL of these cells are diluted 1:20 with Wash buffer (10 µL cells + 190 µL Wash buffer). If cell suspension is too dense, a proper counting cannot be guaranteed. The rest of the cells is kept in case another dilution has to be prepared.

20 µL of diluted cells are added to the BD Trucount-Tube. Meanwhile a Mix with the following antibodies should be prepared: anti-CD3, anti-CD45, 7AAD. Antibodies are diluted 1:100 and 7AAD 1:40 with Wash buffer and 100 µL of this antibody mix is added to the BD Trucount tube. Afterwards the sample should be vortexed and incubated for 15 min at 4°C in the dark. After incubation, 100 µL Wash buffer is added to the sample and the sample is acquired on the flow cytometer. Analysis of results is to be performed using Flow Jo Software. Total counts of CD45 CD3 double positive and viable cells should be evaluated.

If cell count on day 7 is below 0.5×10⁶ viable CD3 positive cells, transition to Expansion Process will not be performed, cell product needs to be destroyed and Pre-Expansion noted as "failed". In addition, the mycoplasma test should be negative to continue with the expansion culture.

### Expansion till Day 14

Depending on the cell count, cells are transferred to a new G-Rex device (0.5 -10×10⁶ viable CD3 positive cells are transferred to a 10M-CS; >10-49.99 ×10⁶ viable CD3 positive cells are transferred to a 50M-CS; ≥50 ×10⁶ viable CD3 positive cells are transferred to a 100M-CS). Therefore, cells are resuspended and applied with a sterile syringe to the new G-Rex device. Afterwards Expansion Medium (AIM complete containing 3000 IU/mL IL-2, 5 ng/mL IL-7, 5 ng/mL IL-15, 5 ng/mL IL-21) is added using the GatheRex device.

The process is controlled via glucose/ lactate measurement on day 10 and 12. If lactate level exceeds 12 mM/L, medium has to be renewed.

On Day 14 consumed medium is collected from the G-Rex device with the GatheRex System and the cells are carefully resuspended in remaining volume.

### Day 14 Flow cytometry for In Process Controls and Release Testing

BD-Trucount tubes are used for counting of cells. Therefore, a small number of cells (50 µL) is taken from the G-Rex System. 10 µL of these cells are diluted 1:20 with Wash buffer (10 µL cells + 190 µL Wash buffer). If cell suspension is too dense, a proper counting cannot be guaranteed. The rest of the cells is kept in case another dilution has to be prepared.

20 µL of diluted cells are added to the BD Trucount-Tube. Meanwhile a Mix with the following antibodies should be prepared: anti-CD3, anti-CD45, 7AAD. Antibodies are diluted 1:100 with Wash buffer and 100 µL of this antibody mix is added to the BD Trucount tube. Afterwards the sample should be vortexed and incubated for 15 min at 4°C in the dark. After incubation, 100 µL Wash buffer is added to the sample and the sample is acquired on the flow cytometer. Analysis of results is to be performed using Flow Jo Software. Total counts of CD45 CD3 double positive viable cells should be evaluated.

If cell count at day 14 is lower than 25×10⁶ viable CD3 positive cells, the process is considered as "failed" and the cells are discarded.

### Expansion till Day 21

Depending on the cell count and specification of G-Rex devices, cells are either transferred to a larger G-Rex Device or kept in the current one.

For transferring the cells to a new G-Rex device the GatheRex system is used. When cells have been transferred, new Expansion Medium (AIM V complete containing 3000 IU/mL IL-2, 5 ng/mL IL-7, 5 ng/mL IL-15, 5 ng/mL IL-21) is added via the GatheRex system. If cells are kept in the current G-Rex device, new Expansion Medium (AIM V complete containing 3000 IU/mL IL-2, 5 ng/mL IL-7, 5 ng/mL IL-15, 5 ng/mL IL-21) is added via the GatheRex system. The process is controlled via glucose/ lactate measurement on day16, 18 and 19. If lactate level exceeds 12 mM/L, medium has to be renewed.

On day 21 cells are harvested. Therefore, a collection bag has to be weighted and weight (tara) has to be documented in the batch manufacturing record. The collection bag and a waste bag are welded to the G-Rex device. Consumed medium is collected in the waste bag with the GatheRex device. Subsequently the cells are resuspended in the remaining volume and transferred to the collection bag. To collect any remaining cells, a small amount of used medium is rinsed back into the G-Rex device and then transferred to the collection bag via the GatheRex system. Afterwards the collection bag has to be weighted. The total volume is the difference between the weighed bag and the empty bag (tara).

For cell count a C-Pro sampling line is sterilely connected to the collection bag and <1 mL sample is collected. From this sample the mycoplasma test should be performed.

### Day 21 Flow cytometry for In Process Controls and Release Testing

10 µL of these cells are diluted 1:20 with Wash buffer (10 µL cells + 190 µL Wash buffer). If cell suspension is too dense, a proper counting cannot be guaranteed. The rest of the cells is kept in case another dilution has to be done.

20 µL of diluted cells are added to the BD Trucount-Tube. Meanwhile a Mix with the following antibodies should be prepared: anti-CD3, anti-CD45, 7AAD. Antibodies are diluted 1:100 with Wash buffer and 100 µL of this antibody mix is added to the BD Trucount tube. Afterwards the sample should be vortexed and incubated for 15 min at 4°C in the dark. After incubation, 100 µL

Wash buffer is added to the sample and the sample is acquired on the flow cytometer. Analysis of results is to be performed with Flow Jo Software. Total counts of CD45 CD3 double positive viable cells should be evaluated.

### Modification of xpsTILS

In this process steps the cultivated TILS are rebuffered in electroporation buffer, modified with siRNA by electroporation and afterwards finally formulated.

### xpsTIL rebuffering

For the process of rebuffering the CTS Rotea Counterflow Centrifugation System is used. In brief, 1.5 ×10⁹ cells are transferred to a new 250 mL bag (= Source bag). Afterwards the source bag, waste bag, electroporation buffer bag, sample bag (from the MaxCyte electroporation kit) are sterilely welded to a Rotea kit. Then the Rotea kit is fitted into the Rotea Counterflow Centrifuge. For set up kit installation, a general quick start guide is provided by Thermo Fisher. The harvest volume should be 15 mL. The Lovo^{®} device from Fresenius Kabi or other devices known from the state of the art can be used as alternative devices.

### xpsTILS modification

For the following manufacturing step the MaxCyte^{®} ExPERT GTx system is used. A vial containing 1 mL of cbl-b specific siRNA has to be thawed on room temperature. Meanwhile the sample bag containing 15 mL TILS in electroporation buffer is transferred to a class A laminar air flow hood. 1 mL sample (S01) is drawn aseptically using a sterile syringe. 50 µL of these cells are transferred to a sterile 1.5 mL tube for cell count with BD TruCount. The remaining cells are stored at room temperature until completion of the manufacturing.

Then the thawed siRNA (1 mL) is added to the cell preparation bag with a syringe using a safety cannula and the designated syringe sterile filter. The bag is reconnected to the MaxCyte^{®} processing assembly kit and is fitted in the MaxCyte^{®} ExPERT GTx device. For electroporation the application 'Expanded T-Cell 3' is started.

After electroporation cells are stored at room temperature for 30-60min.

### xpsTIL formulation

The process of formulation is performed with the CTS Rotea Counterflow Centrifugation System.

After 30-60 min storage of the electroporated cells the sample bag (source bag), a waste bag, a bag containing Plasmalyte and a harvest bag are sterilely welded to a Rotea single use kit. The Rotea kit is fitted into the Rotea Counterflow Centrifuge and the protocol "INV441_DP formulation" is started in the corresponding Rotea software. The drug product is finally formulated in Plasmalyte with a volume of 10 mL.

For release testing 5 mL of sample is transferred to a Cytiva C-Pro sepax sampling line. This sample is referred to as Sample "S02".

4 mL of S02 is collected with a syringe for the sterility testing with the BactAlert System.

1 mL is transferred to a sterile 15 mL tube for release testing:
900 µL for release testing sample generation 50 µL for endotoxin testing
50 µL for BD TruCount cell count

### Flow cytometry for In Process Controls and Release Testing

BD TruCount analysis is performed to determine the cell count of NPC and Drug product.

Since the cell suspension of S01 and S02 is too dense to directly perform the BD TruCount measurement, S01 and S02 should be diluted. Therefore 10 µL are diluted 1:50 with Wash buffer (10 µL cells + 490 µL Wash buffer). The rest of the cells is kept in case another dilution has to be performed.

20 µL of diluted S01 and S02 are added to respective BD Trucount-Tubes. Meanwhile a Mix with the following antibodies should be prepared: anti-CD3, anti-CD45, 7AAD. Antibodies are diluted 1:100 with Wash buffer and 100 µL of the mix is applied to each of the BD Trucount tubes. Afterwards the samples should be vortexed and incubated for 15 min at 4°C in the dark. After incubation, 100 µL Wash buffer is added to each sample and the sample is acquired on the flow cytometer. Analysis of results is to be performed with Flow Jo Software. Total counts of CD45 CD3 double positive viable cells should be evaluated.

Total count of S01 is relevant for sample preparation release testing. Total count of S02 is relevant for final formulation of drug product but also for sample preparation release testing.

### Release Testing

As release testing parameter sterility, visual inspection, endotoxin testing, mycoplasma testing, activity and potency testing are performed according to manufacturer's instruction.

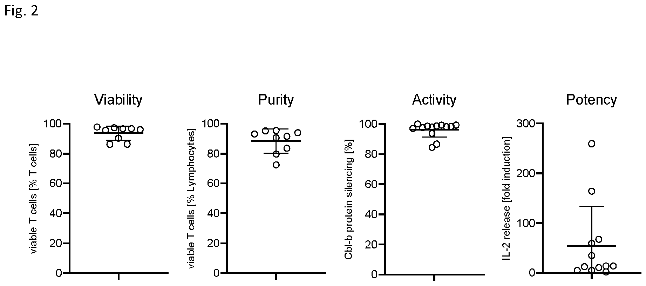

Figure 2 depicts the results for viability, purify, activity and potency of Cbl-b silenced TILs. Viability (percent live cells of CD3 T cells), purity (percent live T cells of lymphocytes), activity (Cbl-b protein silencing in S02 compared to S01/NPC) and potency (IL-2 release S02 fold S01) of Cbl-b silenced TILs are shown in Figure 2.

### Example 2: Materials and Methods

### 2.1 PBMC isolation from CPT tubes

PBMCs from healthy donors were isolated from Vacutainer^{®} CPT^{™} tubes (362782, Becton Dickinson) according to manufacturer's instructions. In brief, whole blood was collected into Vacutainer CPT tubes, inverted, and centrifuged at room temperature (RT) with brakes switched on. Plasma was discarded, leukocytes were harvested, washed with PBS and counted using a hematology analyzer for complete blood counts (CBC; Hematology Analyzer DxH500, Beckman Coulter Diagnostics).

### 2.2 PBMC isolation from buffy coats

Buffy coats were purchased from the Austrian Red Cross blood donation center. PBMCs were isolated from buffy coats according to manufacturer's instructions using Lymphoprep^{™} (StemCell Technologies). In brief, buffy coats were diluted with PBS, under-layed with Lymphoprep^{™} and centrifuged with brakes switched off. The resulting interphase containing lymphocytes was harvested and washed with PBS. Cell pellets were pooled and washed twice with PBS. The resulting cell pellet was resuspended in PBS and cells were counted using a hematology analyzer for complete blood counts (CBC; Hematology Analyzer DxH500, Beckman Coulter Diagnostics).

### 2.3. PBMC isolation from EDTA Vacutainer^{®} tubes

PBMCs from Glioblastoma patients were isolated from Vacuette^{®} EDTA tubes (456036, Greiner Bio-one) according to Lymphoprep manufacturer's instructions. In brief, whole blood was collected into Vacutainer EDTA tubes for transport. Cells were counted for complete blood counts (CBC; Hematology Analyzer DxH 520, Beckman Coulter Diagnostics). 500 µl of blood were transferred to 1.5 mL tube to generate plasma (250 µl, stored at - 80°C). PBMCs were isolated using Lymphoprep (Stemcell Technologies). Cells were counted, analyzed by flow cytometry (*immune landscape* panel, see 2.8) and frozen as cell pellets for TCR Sequencing.

### 2.4. TIL isolation from patient liquor/CSF or tumor tissue

CSF from glioma patients was harvested via their preexisting Ommaya Reservoir or prior to tumor surgery via punctation of the dura mater by qualified personnel from the Department for Neurosurgery of the Medical University Vienna (MUV). Tumor tissue was removed during surgery and placed into sterile PBS. Tumor tissues were digested with a brain tumor digestion kit (130-095-942, Miltenyi Biotec) or Tumor Dissociation Kit (Patient 10, 130-095-929, Miltenyi Biotec) according to manufacturer's instructions using C-tubes (Miltenyi Biotec) in a gentleMACS Octo Dissociator (Miltenyi Biotec, protocol 37C_BTDK_1).

### 2.5. Expansion and phenotypic shift (xps) of T cells or TILs in G-Rex bioreactors

Incoming starting material was analyzed by flow cytometry (FC) for immune cells in general (*immune landscape* panel, see 2.8). 1-5×10⁶ cells (containing 2×10³ - 2×10⁵ CD3⁺ cells) of the remaining material were directly transferred into a G-Rex 24-well plate (2 cm², 0.5-1×10⁶ cells per cm²) in 2 mL *growth medium* (AIM V, 12055091, Gibco; 2% Physiologix XF SR, 0463-01-001 Nucleus Biologics OR 2% CTS SR, A2596101, Thermo Fisher Scientific; ; 20 mM HEPES, 15630-056 Gibco; 0.05 mM 2-Mercaptoethanol, 21985023 Gibco; 0.5 mM L-arginine, 11009 Sigma; 2 mM Glutamax, 35050-061 Gibco) containing 1× T Cell TransAct (20 µl in 2 mL *expansion medium*, 130-111-160, Miltenyi Biotec). After 24h of activation, 6 mL *expansion medium* were added containing *expansion cytokines* (for 8 mL in total): IL-2 (589106, BioLegend, 3000 IU/ml), IL-7 (581904, BioLegend, 5 ng/ml), IL-15 (570304, BioLegend, 5 ng/ml), and IL-21 (571204, BioLegend, 5 ng/ml). Glucose and lactate levels were measured every 2-3 days (Super GL compact, Hitado GmbH) and medium was renewed when lactate reached a concentration of > 15 mM. Cells were counted every 3-4 days starting from day 7 and were transferred to larger bioreactors in *expansion medium* supplemented with *expansion cytokines* to maintain a target cell density suitable for the used bioreactor (see **Table 1** for cell densities).

**Table 1: G-Rex plates and bioreactors used for TIL expansion and their suggested cell densities for culture.**

| | **24 Well Plate** | **5M** | **6 Well Plate** | **6M Well Plate** | **10M** | **50M** | **100M** |
|---|---|---|---|---|---|---|---|
| surface area (cm²) | 2 | 5 | 10 | 10 | 10 | 50 | 100 |
| starting cell density (×10⁶) | 1 | 2,5 | 5 | 5 | 5 | 25 | 50 |
| liquid for activation (mL) | 2 | 2,5 | 5 | 5 | 10 | 25 | 50 |
| liquid capacity total (mL) | 8 | 50 | 40 | 100 | 110 | 500 | 1000 |
| cell density max (×10⁸) | 0.6-0.8 | 1-2 | 3-4 | 3-4 | 3-4 | 10-20 | 20-40 |

### 2.6. Electroporation of expanded and phenotypic shifted T cells or TILs

Expanded and phenotypic shifted T cells or TILs were silenced for Cbl-b expression by electroporation with siRNA specific for Cbl-b according to WO 2023/237764 A1. In brief, expanded T cells or TILs were counted, washed with PBS, resuspended in electroporation buffer (Miltenyi Biotec) and mixed with 6 µM of siRNA. Electroporation was performed using the CliniMACS electroporator (Miltenyi Biotec) for all experiments using cell expansion in dishes and flasks.

Electroporation of T cells or TILs that were expanded in closed systems (cell expansion bags or G-Rex bioreactors) was performed on an ExPERT GTx^{™} (MaxCyte^{®}) electroporator. Therefore, the bags or G-Rex bioreactors were sterilely welded to a CTS Rotea closed system single-use kits (ThermoFisher Scientific) and rebuffered into electroporation buffer (MaxCyte^{®}) to reach a cell concentration of 120 × 10⁶/mL. The bag containing cells in electroporation buffer was then connected to the CL-2 GMP flow electroporation set (MaxCyte^{®}) by sterile welding. 0.5-1 mL of siRNA (depending on cell number) was injected into the bag via a 0.022 µm filter connected to a Luer connector. Electroporation was performed in 3 mL steps. After electroporation, the bag containing cells was connected to a second Rotea Set (ThermoFisher Scientific) by sterile welding and cells were rebuffered for final formulation (Plasmalyte, 4E0324, Baxter or Plasmalyte containing 2 % HSA) at a final concentration of more than 30 × 10⁶ cells per mL.

### 2.7. Cytotoxicity assay

Cytotoxicity was measured using a xCELLigence RTCA (Agilent Technologies) or Maestro TrayZ (Axion Biosystems). On the day before performing the cytotoxicity co-culture assay, M21 melanoma cells were seeded on 96-well plates in their culture medium on fibronectin-coated E-plates (10 pg/mL fibronectin; 5,000 cells per 96-well) and incubated overnight at 37°C / 5% CO₂. The next day, expanded T cells or TILS were added to the tumor cells at different effector:target (E:T) ratios. Tumor cell growth was recorded by impedance measurements for up to 60 h from start of tumor cells-T cell/TIL co-culture.

### 2.8 ELISA

ELISA for cytokines IL-2, IL-15, IL-7, and IL-21 were purchased from RnD Systems (DY207, IL-7) or eBioscience (88-8218-88, IL-21) or BD (555190, IL-2; 559268, IL-15). Supernatant samples were diluted with PBS and analyzed according to manufacturer's instructions.

### 2.9. Flow Cytometry

For analysis via Flow Cytometry the following antibodies were used:
Immune landscape: fix.viability-eF780, CD45-BV421, CD3-AF700, CD14-APC, CD56-PE, CD19-PE-Cy7, CD11b-BV711, CD15-PerCP-Cy5.5, CD33-BV785, HLA-DR-KIRAVIA
T cell memory panel: fix.viability-eF780, CD3-AF700, CD4-BUV395, CD8-BUV496, CD56-BV421, CD45RA-BV711, CD45RO-APC, CD62L-BV785, CCR7-PE-Cy7, CD27-PE, FAS/CD95-FITC
T cell activation panel: fix.viability-eF780, CD3-AF700, CD4-BUV395, CD8-BUV496, CD69-BV421, HLA-DR-KIRAVIA, CD25-PE, CD71-APC
Immune checkpoint panel: fix.viability-eF780, CD3-AF700, CD4-BUV395, CD8-BUV496, PD1-FITC, CTLA-4-PE, TIM3-APC, LAG3-BV421, TIGIT-BV785
T cell cytokine receptor panel: fix.viability-eF780, CD3-AF700, CD4-BUV395, CD8-BUV496, TCRγδ-APC, CD25-BV785, CD127-FITC, CD215-PE, CD360-BV421
T cell regulatory panel: fix.viability-eF780, CD3-AF700, CD4-BUV395, CD8-BUV496, CD25-BV421, Foxp3-AF488
T cell proliferation panel: fix.viability-eF780, CD3-AF700, CD4-BUV395, CD8-BUV496, CellTrace^{™} Violet Dye (Invitrogen)
0.2×10⁶ cells were stained in 50 µl FACS Buffer (PBS, 5% FCS (Gibco, 10500064), 2 mM EDTA for 15 min. Samples were acquired on a CytoFLEX LX Flow Cytometer (Beckman Coulter).

For intracellular detection of FoxP3 True-Nuclear Transcription Factor Buffer Set (BioLegend) was used according to manufacturer's instructions.

For analysis of proliferation cells were labeled with 5 µM CellTrace^{™} Violet Dye (CTV, Invitrogen, 10220455).

### 2.10. Detection of lactate and glucose

Determination of lactate and glucose concentrations were performed every 2-3 days with Super GL compact (Hitado GmbH) according to manufacturer's instructions.

### 2.11. qRT-PCR

Determination of Cbl-b and FOXP3 expression expanded lymphocytes was performed by real time reverse transcription polymerase chain reaction (RT-PCR). RNA was isolated from anti-CD3/CD28 stimulated no pulse control and drug product samples using the Monarch^{®} Total RNA Miniprep Kit (#T2010S, New England Biolabs) according to manufacturer's protocol. Cbl-b and FOXP3 expression was shown in relation to the reference housekeeping gene EF1α. For this purpose, specific primer/probe mixes for EF1α (Hs00265885_g1, Thermo Fisher Scientific) Cbl-b (Hs00180288_m1, Thermo Fisher Scientific) and FOXP3 (Hs01085834_m1, Thermo Fisher Scientific) in combination with the Superscript^{®} III Platinum^{®} One-Step quantitative RT-PCR Kit (11732-088, Invitrogen) were used. cDNA Synthesis was performed at 48°C for 30 minutes and RT was deactivated at 95°C for five minutes following 40 cycles of PCR amplification (15 seconds 95°C and one minute 60°C, ramp rate: 1.6°C/s). Fluorescence signal was detected after each cycle. Cbl-b/FOXP3 expression was calculated as ΔΔCt value: [(Ct EF1α - Ct Cbl-b)² = ΔΔCt]. Gene expression was shown as percentage - sample in relation to a reference housekeeping gene.

### 2.12. Western blot

For protein analysis via Western Blotting, cells were harvested, snap frozen in liquid nitrogen and stored at -20°C or - 80°C until further analysis. Cell pellets were thawed on ice and lysed using Cell Lysis Buffer (CST, #9803) with added Protease/Phosphatase Inhibitor Cocktail (CST, #5872S). Protein concentrations of cell lysates were determined by Bradford assay using BSA Standard (Thermo Scientific^{™}, 23209) and Coomassie Protein Assay Reagent (Thermo Scientific^{™}, 1856209). Cell lysates were separated using NuPAGE TM Bis-Tris protein gels (Invitrogen^{™}, 10780345) with NuPAGE^{™} MOPS SDS Running Buffer (20X) (Invitrogen^{™}, 1771490) and transferred to PVDF membrane (Invitrogen^{™}, 15249296). The following monoclonal primary antibodies were used: Cbl-b (CST, #9498) and anti-GAPDH (BioLegend, 649202). Horseradish peroxidase conjugated Anti-mouse IgG (#7076) and Anti-rabbit IgG (#7074) were purchased from CST and used as secondary antibodies. Detection of chemiluminescence was performed using WesternBright Chemilumineszenz Substrat Quantum (Biozym, K-12042-C20) and Azure c600 Imaging System (Azure Biosystems) .

To quantify cytokines IFN-γ, IL-4, FLT3L, IL-2, GM-CSF, sCD40L, sCD25, IL-7, IL-10, IL-15, Granzyme A, TNF-α, Granzyme B and Granulysin in cell supernatants the customized Legendplex kit from BioLegend was used. Acquisition of the samples was performed with a Cytoflex LX Flow Cytometer (Beckman Coulter) and analysed with Legendplex^{™} Data Analysis Software.

### Example 3: xpsTIL expansion protocol expands T cells

Peripheral T cells from buffy coat-derived PBMCs were used as starting material for a cultivation protocol that allows for specific expansion of T cells. The relative leukocyte populations in expansion cultures over time are shown in **Fehler! Verweisquelle konnte nicht gefunden werden.**3 (* no cell expansion in run#3, § run#6 was expanded in G-Rex 6-well plates). PBMCs from healthy donors on day 0 and cultured cells were analyzed via flow cytometry for various markers for lineage (Fehler! Ver**weisquelle konnte nicht gefunden werden.),** activation **(Fehler! Verweisquelle konnte nicht gefunden werden.),** cytokine receptors **(Fehler! Verweisquelle konnte nicht gefunden werden.**5), immune checkpoints **(Fehler! Verweisquelle konnte nicht gefunden werden.**6), memory phenotype **(Fehler! Verweisquelle konnte nicht gefunden werden.**7) on day 7, day 14 and day 21.

### Example 4: Expression of Cbl-b in T cells during expansion

Cbl-b mRNA and Cbl-b protein expression was assessed by qRT-PCR and western blotting, respectively. In both assays Cbl-b was detected. Western blotting showed that Cbl-b protein was undetectable in freshly isolated PBMCs but was expressed at low levels after 14 and 21 days of T cell expansion. Upon anti-CD3 or anti-CD3/CD28 stimulation Cbl-b mRNA and protein expression were increased. Cbl-b mRNA and protein was detected during expansion culture of TILs even in non-stimulated cells. In further experiments, Cbl-b was silenced via siRNA-mediated mRNA degradation.

### Example 5: Expenditure of cytokines used for T cell expansion

Levels of cytokines for T cell expansion were measured in the medium to assess their expenditure over time and adjust dosing if necessary. The cytokines used for expansion were chosen considering the following insights: IL-2 improves outgrowth of TILs from tumor tissue and IL-2 together with aCD3-stimulation (using the OKT3 clone) can cause a rapid expansion phase. T cells cultured in IL-2 are shifted towards an effector memory phenotype enabling them to efficiently recognize and kill their targets. Their longevity is rather short compared to memory T cells or even stem-cell like memory T cells. IL-7 and IL-15 help to maintain their phenotype. Experiments using a combination of IL-7 and IL-15 (without IL-2) showed that lack of IL-2 in the expansion culture resulted in low cell yield compared to cultures that contained IL-2. IL-21 was added to the expansion culture based on data showing that CD4 helper T cells needed IL-21 for proliferation. Cellular products for immunotherapy were further shown to be more efficient when at least a low amount of helper T cells were present compared to pure CD8 cytotoxic T cell products.

### Example 6: Immunologic properties of expanded T cells

Expanded T cells were stimulated with anti-CD3 or anti-CD3/CD28 to induce the production of the cytokines IL-2 and IFN -γ on different days of expansion to assess their immunologic potency. The amount of produced IL-2 and IFN-γ increased during culture time suggesting that the immunological potency would be higher in cells cultured for 2-3 weeks compared to shorter term culture.

### Example 7: Proliferative capacity of expanded T cells

Expanded T cells were assessed for their proliferative capacity during expansion to assess whether their fitness changed during the time of culture. T cells cultured for 21 days retained their proliferative capacity using this in vitro method. Nevertheless, T cells continued to proliferate during their expansion culture and increased in number showing that these cells did not become exhausted or anergic.

### Example 8: In vitro cytotoxic potency of expanded T cells

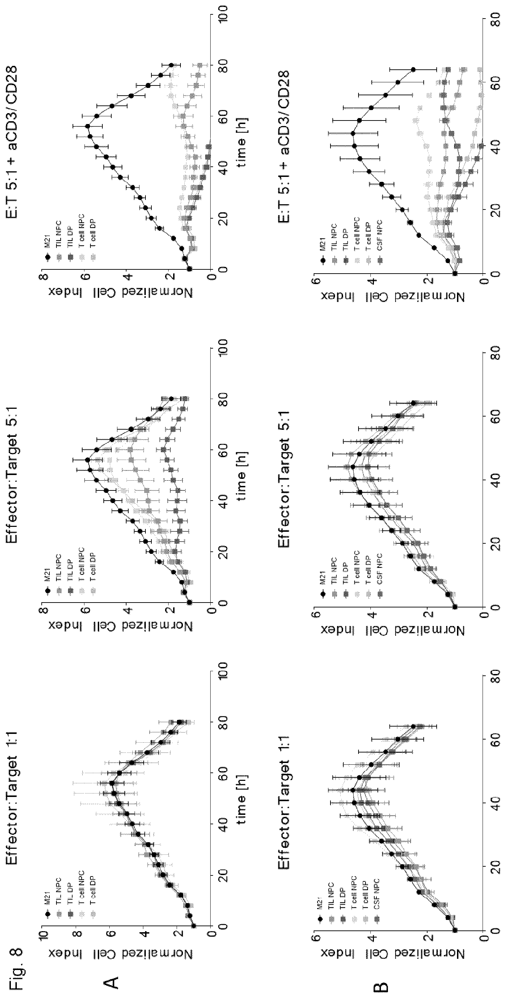

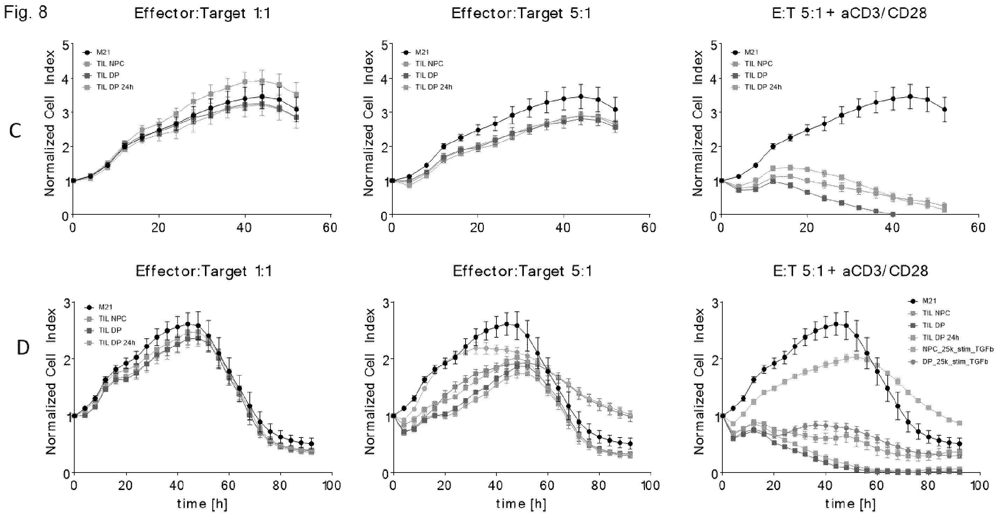

*In vitro* expanded TILs were tested for anti-tumor activity in co-culture with human M21 melanoma cells in order to assess their tumor killing capacity during expansion. Figure 8 depicts in vitro cytotoxicity of patient 11 (A), patient 15 (B), patient 16 (C), or patient 17 (D) derived T cells or TILs silenced (drug product, DP) or not silenced (no-pulse control, NPC) for Cbl-b co-cultured in different effector-to-target ratios with M21 melanoma cells in presence or absence of additional TCR-stimulation (aCD3/CD28) and TFG-β.

### Example 9: Viability and purity of expanded T cells

Viability (% live of CD3+CD45+) and purity (% live CD3 of lymphocyte gate) of expanded T cells were measured after 21 days of culture using flow cytometry. In general, viability and purity of all expanded cells after 3 weeks of culture were above 80%. Furthermore, the ratio of CD4 helper T cells to CD8 cytotoxic T cells (CD4/CD8 ratio) and the percentage of non-exhausted (negative for PD1, Lag3 and Tim3) TILs was analyzed. The CD4/CD8 ratio varied between 0.4 and 2 with values between 0.4 and 0.8 being most common. Overall 60% to 80% of TILS were non-exhausted.

### Example 10: Re-buffering of expanded T cells for Cbl-b-silencing and cytotoxic potency

Expanded T cells from healthy donors or patient PBMCs as well as Glioblastoma tissue TILs were rebuffered into electroporation buffer (MaxCyte^{®}) using the Rotea counterflow centrifuge (Thermo Fisher Scientific). Cbl-b-silencing was done via electroporation as described in WO 2023/237764 A1.

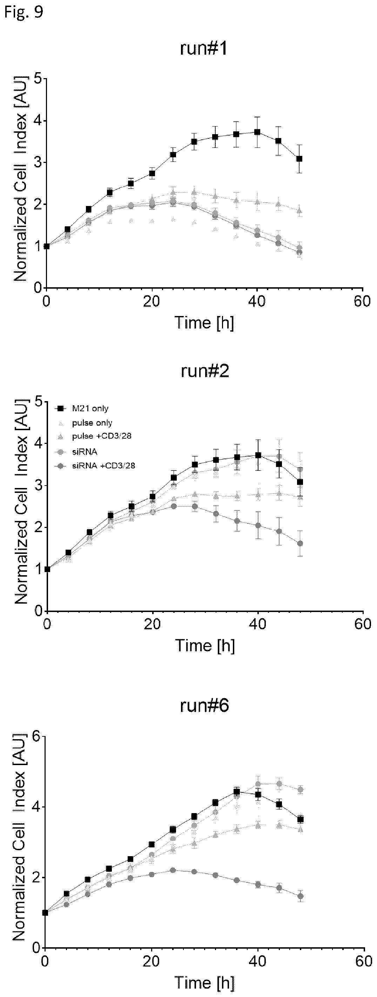

Anti-tumor cytotoxicity of expanded and electroporated T cells was assessed after resting for 24h in stimulating cytokines. After this resting phase, co-culture with human M21 melanoma cells was started and tumor cell growth measured on the xCELLigence RTCA (Agilent Technologies) system in the presence or absence of anti-CD3/CD28 stimulation (Figure 9: Run#1 and #2 were expanded in bags, run#6 was expanded in G-Rex bioreactors.). Expanded and Cbl-b silenced TILs show strong tumor cytotoxicity in vitro and increased anti-tumor cytotoxicity compared to non-modified TILs and to expanded, silenced and non-silenced T cells (NPC).

### Example 11: Preclinical proof of concept in murine glioblastoma models

The purpose of the experiments is to provide preclinical proof-of-concept for the treatment of glioblastoma multiforme (GBM) with Cbl-b-deficient, in vitro expanded tumor infiltrating lymphocytes (TILs). Two different syngeneic GBM cell lines were injected into Cbl-b^{-/-} animals and corresponding WT controls. In addition, in vitro expanded TILs from these tumors were adoptively transferred into tumor-bearing wildtype (WT) recipients to evaluate whether this type of immunotherapy would provide a clinical benefit.

In summary, Cbl-b-deficient animals were able to control the growth of subcutaneously and intracranially grown GBM tumors. Furthermore, intracranial transfer of in vitro expanded Cbl-b^{-/-} TILs induced tumor control and prolonged survival of GL261-tumor-bearing WT mice. These data suggest a clinical benefit of cbl-b modified TIL immunotherapy for the treatment of glioblastoma multiforme.

### MATERIALS AND METHODS

### Cell lines

GL261 (no. ACC 802) and SB28 Ohlfest cells (no. ACC 880, GFP+, Luciferase+) were purchased from DSMZ (German Collection of Microorganisms and Cell Cultures GmbH) and cultured in DMEM (high glucose), 10 % FCS, 1 % PenStrep, 1 % glutamine at 37°C and 5 % CO₂ in a humidified incubator.

SB28 cells were cultured in medium without additional glutamine. Cells were grown to 70-80 % confluency before harvest for in vivo experiments. For intracranial injections, GL261-Luc2-RFP670 were used.

### In vivo experiments

For s.c. GBM injection 6 to 10-week-old male and female mice on C57BL/6J background were used.

The animals were bred and housed under specific pathogen-free (SPF) conditions in a 12h light:12h dark cycle. Corresponding WT controls were used as controls. 2 or 5×10⁶ GL261 GBM cells or 1×10⁶ SB28 Ohlfest were resuspended in 25 µl PBS, mixed with equal volumes of Matrigel (Corning) and kept on ice until used for subcutaneous (s.c.) injection. Tumor size was measured with a caliper and volume was calculated according to the formula: volume = (Dxd²)/2, where D represents the longest diameter and d the shorter, perpendicular diameter of the tumor.

For intracranial (i.c.) injection 12-week-old male and female mice on C57BL/6J background (Cbl-b^{-/-} or corresponding WT controls) were used. The animals were anesthetized via intraperitoneal injection with Ketamin/Xylazin mixture (Ketamin 100 mg/kg, Xylazin 12 mg/kg). Injections were performed using a stereotactic apparatus (model 1900, David Kopf instruments) while keeping the animal's head in a fixed position. During the operation the body temperature was kept at 36°C and controlled using a rectal thermometer.

The skin of the fixed head was anesthetized locally (0.1 mL Xylanaest ^{®} 1 % s.c., Gebro Pharma), the skin was opened anterior-posterior to reveal the skull and drilled into it with a stereoscopic drill (model 1911, David Kopf instruments). Tumor cells were injected at anterior-posterior -1.0 mm (from Bregma), medial-lateral 2.0 mm (from midline), dorsal-ventral 3.0 mm using a Micro4 Syringe Pump Controller (World Precision Instruments) at a flow rate of 4 nl/sec up to 2000 nl in total.

Injections were followed by a 5 min resting time before retracting the needle to prevent backflow of cells. Wounds were sealed with Silkam^{®}. After recovery from anesthesia animals were put back into their cages. Animals were provided with analgesics (Rimadyl^{®}, Carprofen) and antibiotics (Baytril^{®} enrofloxacin) ad libitum via the drinking water starting one day prior GBM cell injection.

### TIL and T cell in vitro expansion

Whole brains from intracranially injected mice were cut into small (2-5 mm³) pieces and digested using a mixture of DNAse (0.09 mg/brain; Roche, 11284932001), Papain (3 mg/brain; Worthington, 35A15522) and collagenase/dispase (1.5 mg/brain; Sigma Aldrich 11097113001) in 3 mL DMEM in GentleMACS C-tubes (Miltenyi Biotec) using a GentleMACS OctoDissociator (Miltenyi Biotec, protocol 37C_ABDK_01). Dissociated and digested cells were filtered through a 70 µm cell strainer and resuspended in 37 % Percoll/HBSS. Cells were transferred to a 50 mL tube and underlaid with 70 % Percoll/HBSS. Subsequently the mixture was overlaid with 30 % Percoll/HBSS and then 1x HBSS.

After centrifugation (without brakes) the 70-37 % interphase was collected, cells were washed with HBSS and used for in vitro expansion.

Subcutaneous tumors were excised, cut into 2-4 mm³ pieces and digested using the Tumor Dissociation Kit for mouse (130-096-730, Miltenyi Biotec) according to manufacturer's instructions. For some experiments tumor draining lymph nodes (TdLN) were used as starting material for T cell expansion. Lymph nodes were excised, forced through a 70 µm cell strainer, washed in medium (RPMI, 10 % FCS, 1 % PenStrep) and used for in vitro expansion of T cells.

Subcutaneous tumor cell suspensions, Percoll-purified brain-derived leukocytes or whole lymph node cells were incubated overnight on dishes precoated with anti-CD3 (2 pg/ml; clone 17A2; BioLegend) and anti-CD28 (1 pg/ml; clone 37.51; BioLegend) in TexMACS 5 % FCS, 20 mM HEPES, 50 µM beta-mercaptoethanol, 1 % PenStrep, 2 mM GlutaMAX, 500 µM L-arginine in a humidified incubator with5 % CO₂. On the next day, the cells were removed from the precoated plates and supplemented with 3000 IU/mL IL-2 (BioLegend, 575406), 5 ng/mL IL-7 (BioLegend, 577804) and 5 ng/mL IL-15(BioLegend, 566302). Medium was renewed every 3-4 days and expanded T cells were used for adoptive cell transfer (ACT) on days 6 or 7. For ACT, cells were resuspended in PBS (100 µl for subcutaneous tumors or 2 µl for intracranial tumors per animal) and injected into the lateral tail vein for subcutaneous tumors or directly into the tumor in intracranial experiments.

### In vitro cytotoxicity assay.

On day 0, GL261 or SB28 cells were seeded in their respective culture medium on fibronectin-coated E-plates (10 µg/mL fibronectin, 20.000 cells/well GL261 or 2.500 cells/well SB28) in 100 µl and incubated overnight at 37°C/5% CO₂. T cells from tumors or tumor draining lymph nodes (TdLN) were expanded for 6-7 days and then used for in vitro cytotoxicity. On day 1 post tumor cell seeding, expanded T cells were added to the tumor cells at different effector:target (E:T) ratios and further incubated for up to 60 h. Cell growth was recorded every 15 min using the xCELLigence RTCA device (Agilent Technologies).

### RESULTS AND DISCUSSION

### Subcutaneous GBM models in Cbl-b^{-/}⁻ mice

Murine GBM tumor cells (GL261) were subcutaneously injected in Cbl-b^{-/-} mice and WT controls. Tumor growth was assessed by caliper measurement starting from day 3 or 4 post tumor cell injection.

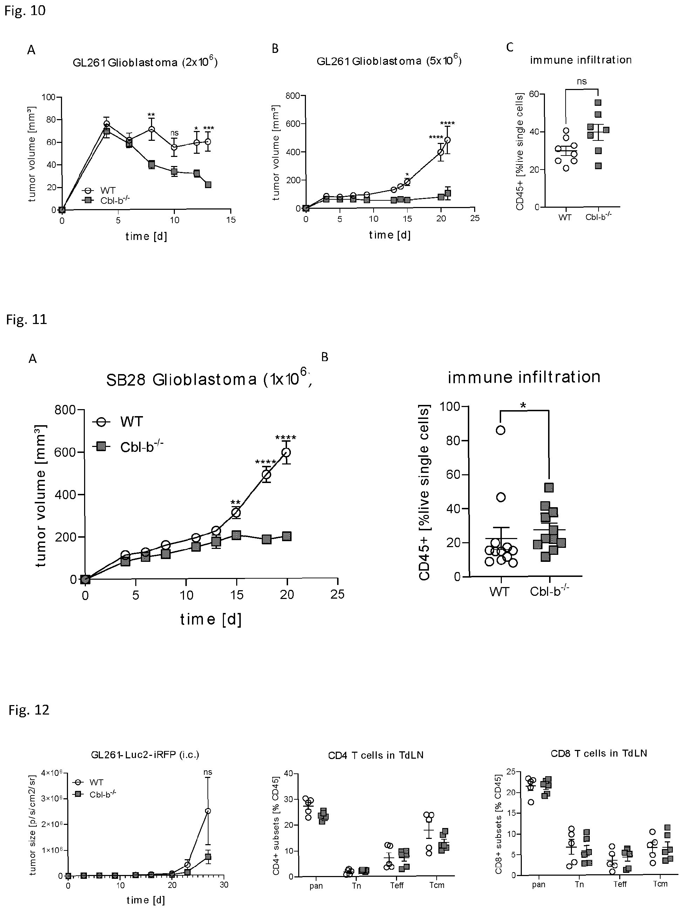

Figure 10 shows the tumor volume in both genotypes when injected with 2×10⁶ or 5×10⁶ GL261 cells.

Cbl-b^{-/-} and WT control mice were injected with 2×10⁶ (A) or 5×10⁶ (B) syngeneic GL261 tumor cells in 50 µl of PBS:Matrigel 1:1. Tumor size was measured starting from days 3-4 post tumor cell injection and volume was calculated as stated in the Methods Section. (C) leukocyte infiltration into GL261 tumors shown in (B) assessed via flow cytometry and expressed as CD45+ cells in percent of all live single cells. (A) n= 5-6 animals per group, (B and C) n= 7-8 animals per group, 2-way ANOVA with Sidak's multiple comparison, ns - not significant, * p<0.05, ** p<0.01, *** p<0.001, **** p<0.0001.

Murine GBM tumor cells (SB28) were subcutaneously injected in Cbl-b^{-/-} mice and WT controls. Tumor growth was assessed by caliper measurement starting from day 4 post tumor cell injection. Figure 11 shows the tumor volume in both genotypes when injected with 1×10⁶ SB28 cells.

Cbl-b^{-/-}and WT control mice were injected with 1×10⁶ syngeneic SB28 tumor cells in 50 µl of PBS:Matrigel 1:1. (A) Tumor size was measured starting from days 4 post tumor cell injection and volume was calculated as stated in the Methods Section.

(B) leukocyte infiltration into SB28 tumors assessed via flow cytometry and expressed as CD45⁺ cells in percent of all live single cells. (A and B) n=12 animals per group, (A) 2-way ANOVA with Sidak's multiple comparison, ** p<0.01, **** p<0.0001, (B) Mann-Whitney test, * p<0.05.

### Orthotopic GBM models in Cbl-b^{-/}⁻ mice

Murine GBM tumor cells (GL261-Luc2-iRFP or SB28) were intracranially injected in Cbl-b^{-/-} mice and WT controls. Tumor growth was assessed by luciferase measurement on an IVIS Spectrum III (Perkin Elmer) starting from day 3-7 post tumor cell injection. Figure 12 shows the tumor volume in both genotypes when injected with 1×10⁵ GL261-Luc2-iRFP cells.

1×10⁵ Gl261-Luc2-iRFP were intracranially injected into Cbl-b^{-/-} and WT controls. (A) Tumor growth was assessed by luciferase measurement in an IVIS Spectrum III (Perkin Elmer), (B) CD4 T cells and (C) CD8 T cell subsets in mandibular and deep cervical tumor draining lymph nodes (TdLN), n= 5-6 animals per group. (A) 2-way ANOVA with Sidak's multiple comparison, ns - not significant.

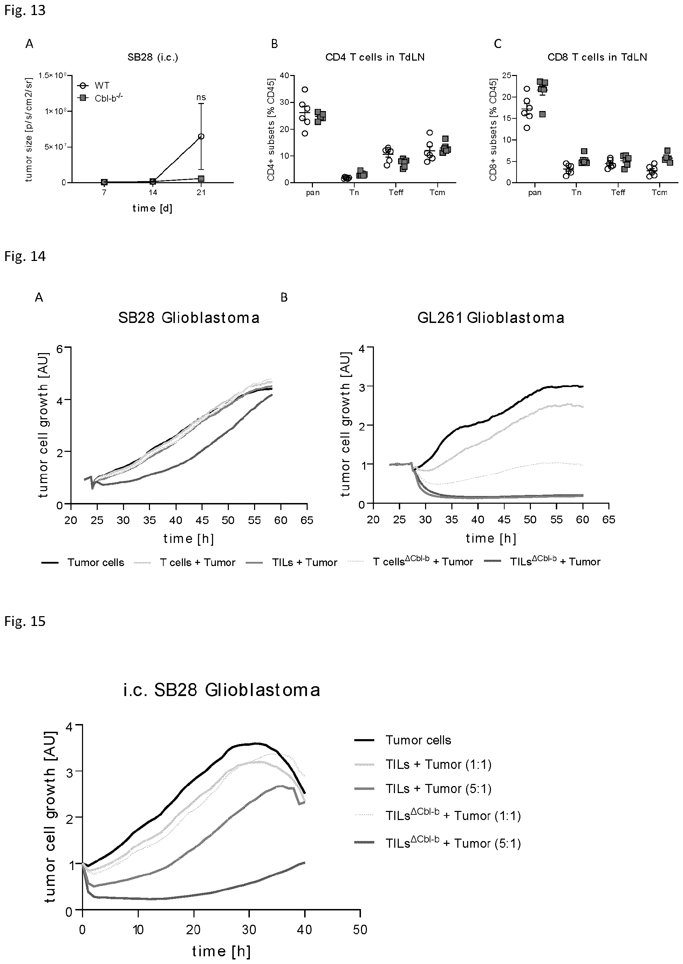

In a second orthotopic tumor model, SB28 cells were intracranially injected in Cbl-b^{-/-} mice and WT controls. Tumor growth was assessed by luciferase measurement on an IVIS Spectrum III (Perkin Elmer) starting from day 7 post tumor cell injection. Figure 13 shows the tumor volume in both genotypes when injected with 2×10³ SB28 cells.

2×10³ SB28 tumor cells were intracranially injected into Cbl-b^{-/-} and WT controls. (A) Tumor growth was assessed by luciferase measurement in an IVIS Spectrum III (Perkin Elmer), (B) CD4 T cells and (C) CD8 T cell subsets in mandibular and deep cervical tumor draining lymph nodes (TdLN), n= 6 animals per group, (A) 2-way ANOVA with Sidak's multiple comparison, ns - not significant.

Cbl-b knock out mice control tumor growth in two immunocompetent glioblastoma models; i.e. tumor growth of s.c. transplanted GL261 and SB28 tumors is inhibited, tumor growth of i.c. transplanted GL261 tumors is delayed and reduced and tumor growth of i.c. transplanted, hardly immuninfiltrated SB28 tumors, closely resembling human glioblastoma is strongly inhibited.

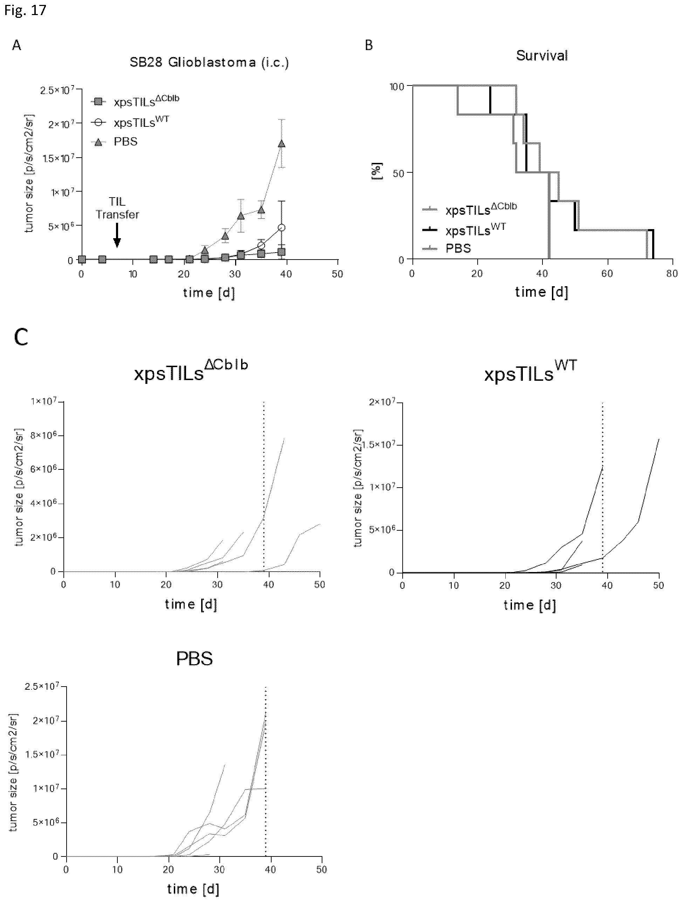

Figure 17 depicts that transfer of expanded Cbl-b^{-/-}(ΔCbl-b) TILs reduces orthotopic SB28 GBM growth and prolongs survival.

### Expanded Cbl-b^{-/}⁻ TILs control GBM tumor growth in vitro

In order to test whether Cbl-b-deficient TILs would be more effective than WT TILs in controlling tumor growth an in vitro cytotoxicity assay using the xCELLigence RTCA device (Agilent Technologies) was set up. GL261 or SB28 cells were seeded on appropriate 96-well plates 24 h before starting the co-culture. The following day, in vitro expanded TILs were added to the tumor cells. Growth of adherent tumor cells (independent of non-adherent T cells) was recorded for more than 48h. Figure 14 shows the growth of SB28 and GL261 cells in co-culture with Cbl-b^{-/-}or WT TILs expanded from subcutaneous tumors and Figure 15 shows co-culture with TILs expanded from orthotopic (i.c.) SB28 tumors.

As shown in Fig 15. expanded WT and Cbl-b^{-/-}(ΔCbl-b) TILs from subcutaneously grown tumors were cocultured with (A) SB28 or (B) GL261 cells in a ratio of 3:1 (T cell to GBM cells) on the xCELLigence RTCA 24 h after initial seeding of the respective tumor cell line. Growth of GBM cells was monitored for up to 60h post initial GBM cell seeding and assessed via impedance measurement.

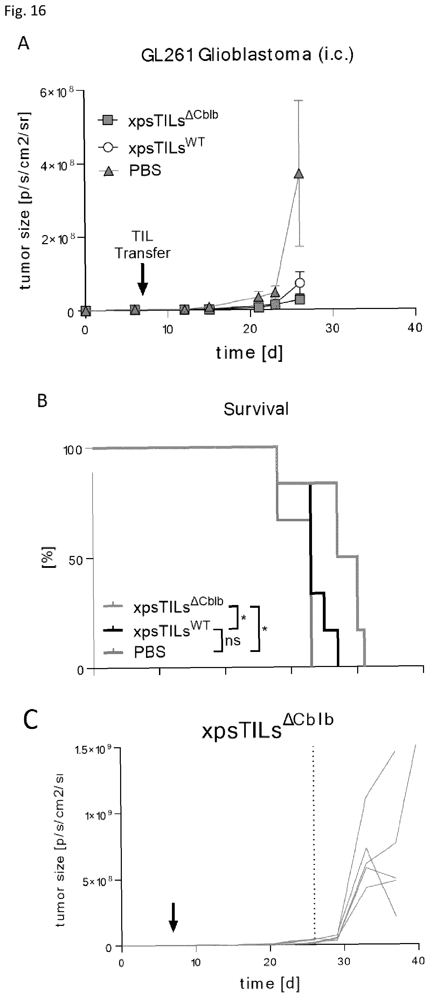

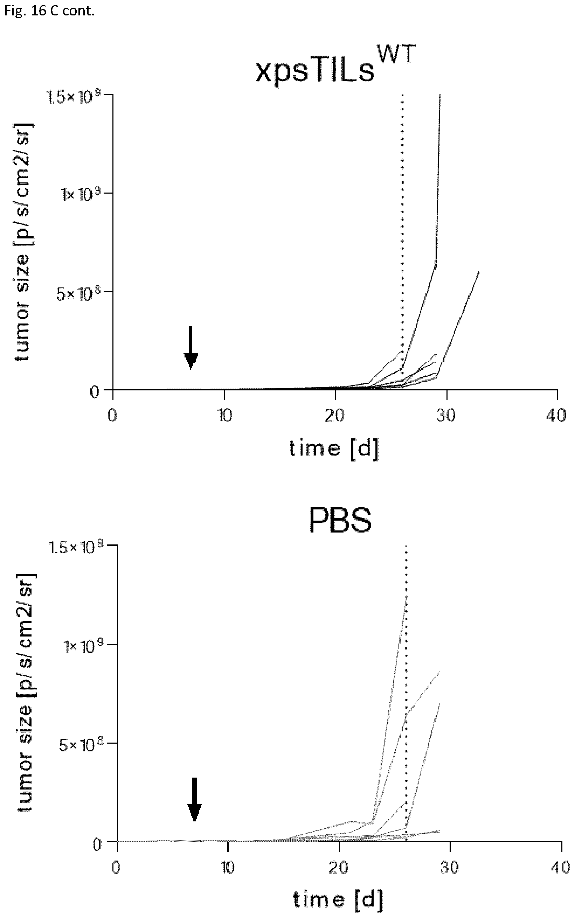

Fig. 16 depicts expanded WT and Cbl-b^{-/-}(ΔCbl-b) TILs from intracranially grown SB28 cocultured with SB28 cells in ratios of 1:1 or 5:1 (TILs to GBM cells) on the xCELLigence RTCA 24 h after initial seeding of the respective tumor cell line. Growth of GBM cells was monitored for up to 40 h post initial GBM cell seeding and assessed via impedance measurement.

Cbl-b knock out TILs show strong killing potential of glioblastoma in vitro, i.e. expanded wildtype TILs (TILsWT) isolated from intracranial (i.c.) SB28 tumors kill SB28 cells in a cell dose dependent manner and in comparison, Cbl-b knock out TILs (TILsΔCbl-b) show strongly enhanced killing potential of SB28 glioblastoma in vitro.

### Transfer of expanded Cbl-b^{-/}⁻ TILs in i.c. GBM models

The next step was to assess whether expanded Cbl-b^{-/-} TILs would also show increased cytotoxicity in vivo. Therefore, expanded Cbl-b^{-/-}or WT TILs were injected into i.c. tumors 7 days post tumor cell inoculation. Figure 16A shows the mean tumor growth in recipient mice after adoptive transfer (ACT) of expanded TILs (or PBS control injection) for up to 26 days. All animals survived up to this time point.

Figure 16C shows the tumor growth of individual animals separated by group (PBS controls, xpsTIL^{WT} or xpsTIL^{ΔCblb}). The survival of animals that received expanded Cbl-b^{-/-} TILs was significantly increased (38.5 days) compared to both, PBS-treated and xpsTIL^{WT}-treated animals (33 days each, Figure 16B).

Figure 16 depicts in (A) tumor growth of ACT recipient mice assessed by luciferase measurement in an IVIS Spectrum III (Perkin Elmer) up to day 26 when all animals were alive, in(B) survival of mice as shown in (A), in (C) individual tumor growth curves of animals shown in (A) for as long as animals were alive, n=6 animals per group, (B) Log-rank Mantel-Cox test, * p<0.05, ns - not significant.

A survival benefit in murine glioblastoma after i.c. transfer of Cbl-b knock out expanded and shifted TILs is shown: i.e. growth of intracranially (i.c.) transplanted GL261 and SB28 glioblastoma is strongly inhibited after i.c. transfer of Cbl-b knock out TILs (TILs^{ΔCbl-b}), compared to mice treated with non-modified TILs (TILs^{WT}); survival is significantly prolonged after i.c transfer of TIL^{ΔCbl-b} into GL261-bearing mice; and survival benefit after i.c. TIL transfer in hardly immunogenic SB28 glioblastoma model.

### DISCUSSION

Glioblastoma multiforme is the most lethal brain tumor in adults with limited treatment options and no available cure. Since the introduction of fractionated chemoradiotherapy as standard of care no further improvement in prolonging survival rates has been achieved. Immunotherapy approaches are currently being developed for the treatment of brain tumors, but so far neither checkpoint inhibitors, nor CAR T cell therapy or vaccination approaches using dendritic cells (DC) has been approved for clinical use.

It is shown in two different murine GBM models that Cbl-b deficiency enables tumor control during s.c. and i.c. tumor growth. The two models differ in their immunogenicity and response to immunotherapy (GL261 are immunogenic and well infiltrated whereas SB28 are hardly immunogenic and therefore only marginally infiltrated) (Letchuman et al. 2022, Figure 1C and Figure 2B) .

In vitro expanded TILs from Cbl-b^{-/-} mice showed increased cytotoxicity in vitro when co-cultured with either cell line compared to T cells of any genotype or TILs from WT mice. Moreover, adoptive transfer of in vitro expanded Cbl-b^{-/-} TILs into GL261-bearing WT mice resulted in increased tumor growth control compared to TIL ACT or sham injection and significantly prolonged survival of recipient animals.

Taken together the invention shows that Cbl-b deficiency leads to tumor control in two independent GBM tumor models that differ in their immunogenicity and immune infiltration. Furthermore, adoptive transfer of expanded Cbl-b^{-/-} TILs reduces tumor growth and significantly prolongs survival in i.c. GL261 tumor bearing mice suggesting that knock down of Cbl-b in expanded TILs can control tumor growth even in hardly immunogenic GBM tumors.

### Example 12: Expansion of human TILs for clinical studies

The following process describes the cultivation and expansion of human Tumor Infiltrating Lymphocytes (TILs) from human brain tumor tissue. TILs are cultivated in G-Rex^{®} devices in a cell count dependent way. After 24 hours of activation via TransAct^{®}, TILs are further cultivated and expanded ex vivo for 20 days (21 days in total). Cell expansion is divided into two parts: Pre-Expansion and Expansion Process. Pre-Expansion may start in an open system - the G-Rex 24-Well Plates - and is necessary to gain the cell number needed for upscaling to 1×10⁹ cells. The Expansion Process is controlled via glucose/lactate measurement every 2-3 days or cell counts using flow cytometry and media - or G-Rex device changes are performed accordingly.

After tumor digest, the sample is centrifuged, and the Expansion Protocol representing an example of the method of the invention starts. The supernatant is gently removed and the pellet with the cells in resupended in 2 mL AIMV activation medium containing 20µl TransAct^{®} in a tube. Then the cell suspension is transferred into the G-Rex 24-well plate and incubated for 24 hours. After the activation period of one day the appropriate amount of expansion medium containing cytokines is added (see Table 2).

**Table 2: Expansion Medium including Cytokines to be added**

| **Medium / Cytokine** | **GRex 24Well Plate** |
|---|---|
| AIM V complete | 6 mL |
| IL - 2 | 4000 IU/mL |
| IL - 7 | 6.67 ng/mL |
| IL - 15 | 6.67 ng/mL |
| IL - 21 | 6.67 ng/mL |

The concentration of lactate and glucose are checked on day 3 and 5 and depending on the concentration of lactate the expansion medium has to be renewed. When adding fresh AIM V expansion medium the concentration of cytokines may be below the concentrations listed in Table 2 (see Table 3).

**Table 3: Expansion Medium including Cytokines to be added during medium change**

| **Medium / Cytokine** | **GRex 24Well Plate** |
|---|---|
| AIM V complete | 6 mL |
| IL - 2 | 3000 IU/mL |
| IL - 7 | 5 ng/mL |
| IL - 15 | 5 ng/mL |
| IL - 21 | 5 ng/mL |

The cells are cultivated until day 7. Cells need to be transferred to a larger G-Rex device (e.g. G-Rex 10M, G-Rex 50M, G-Rex 100M) on day 7, if cell count via flow cytometry on day 7 of expansion shows more than 0.5×10⁶ viable CD3 positive cells.

Cells are further incubated in the closed G-Rex system at least until day 10 using a sterile fluid path for lactate and glucose measurement. The expansion medium needs to be changed if the lactate level is above 8 mM/L to 12 mM/L.

Cells need to be counted at day 14 and transferred to larger G-Rex devices. If the cell-count on day 14 is above 25×10⁶ cells, cells are split to G-Rex 50M and if the cell count is higher than 50×10⁶ cells, a split to G-Rex 100M is performed. Since the cells are transferred to the G-Rex device it is incubated and the cells are expanded until day 21. Further checks for glucose and lactate concentration are performed on day 16, 18, and 20. The cells are harvested according to manufacturer's instructions.

The cells are further processed while rebuffering the expanded tumor infiltrating lymphocytes (xpsTILs) for electroporation. For rebuffering, the CTS Rotea Counterflow Centrifugation System is used. The cell culture suspension in a G-Rex bioreactor is transferred to a disposable bag and aseptically connected to CTS Rotea Single-Use Kit. During the procedure, the xpsTILs are washed, enriched and rebuffered into electroporation buffer to proceed with electroporation.

In the xpsTILs generated according to the method of the invention Cbl-b expression has been transiently silenced via transfection of Cbl-b specific siRNA. After harvesting and rebuffering xpsTILs cell culture suspension, siRNA will be added and electroporation conducted employing the MaxCyte^{®} ExPERT GTx Transfection System, with optimized parameters for transfection of T-cells. Later, the modified xpsTILs are subjected to a second rebuffering step for purification and rebuffering into final formulation solution for reinfusion.

After electroporation, modified xpsTILs are collected in the target bag of the MaxCyte^{®} processing assembly. This bag is reconnected in a sterile way to a second Rotea single-use kit. During the following procedure, the product is depleted of cell debris and rebuffered into Plasmalyte. Sample S02 for determination of cell count and release testing is taken from this final bag. After determination of the total amount of cells and positively concluded release testing, the required cell dose for patient treatment is administered.

### Example 13: Comparison of Rosenberg Protocol to the method of the invention

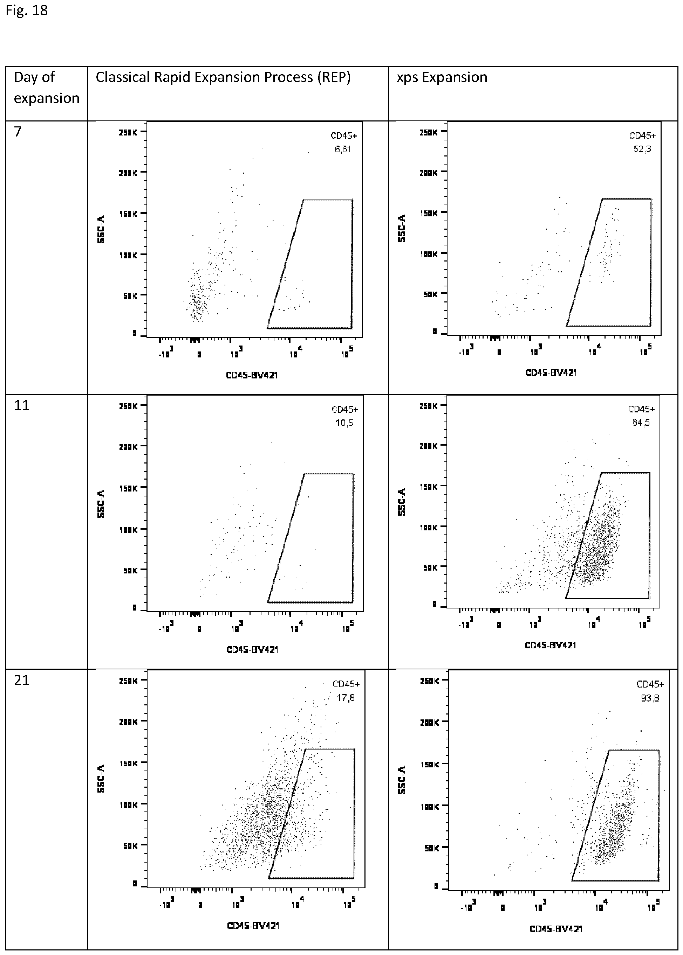

Standard protocols for expansion of TILs use the Rosenberg protocol. It describes that the expansion of TILs from tumor tissue using IL-2 (pre rapid expansion, Pre-REP), anti-CD3 (OKT3) stimulation and feeder cells (for rapid expansion, REP). Therefore, all protocols known from prior art use the Pre-REP and REP protocols. (Jin et al. "Simplified Method of the Growth of Human Tumor in-filtrating Lymphocytes in Gas-permeable Flasks to Numbers Needed for Patient Treatment. J Immunther 2012; 35:283-292. DOI: 10.1097/CJI.0b013e31824e801f and Rosenberg S.A., Spiess P., Lafreniere R. A new approach to the adoptive immunotherapy of cancer with tumor-infiltrating lymphocytes. Science. 1986; 233:1318-1321. doi: 10.1126/science.3489291). In addition, clinical treatment of patients with intravenous TIL infusion require pre-treatment with cyclophosphamide and fludarabine for non-myeloablative lymphodepletion and concomitant high-dose IL-2 infusions to boost i.v. TIL function, accounting for the majority of observed adverse events. Figure 18 shows that CD45 leukocytes can be expanded from glioblastoma tumor tissue with the method of the invention in comparison to the classical rapid expansion protocol (REP) from Rosenberg where the expansion of TILs from glioblastoma tissue was not successful.

## Claims

1. An in-vitro or ex-vivo method of expanding lymphocytes comprising
providing a population of lymphocytes, wherein the lymphocytes are from a biological sample;
culturing the lymphocytes in a growth medium;
expanding the lymphocytes in an expansion medium comprising nutrients and differentiation factors and/or cytokines, wherein the differentiations factors and/or cytokines comprise or consist of interleukin-2, interleukin-7, interleukin-15, and interleukin-21;
obtaining expanded lymphocytes.

2. The method of claim 1, wherein the lymphocytes are T cells and comprise CD4+ T cells and/or CD8+ T cells.

3. The method of claim 1 or 2, wherein the lymphocytes are from a tumor and/or from a fluid, preferably of a tumor; and/or wherein the lymphocytes are tumor infiltrating lymphocytes.

4. The method of claim 3, wherein the tumor is a glioblastoma.

5. The method of any one of claims 1 to 4, wherein the differentiations factors and/or cytokines of the expansion medium do not include interleukin-4 and/or interleukin-17.

6. The method of any one of claims 1 to 5, wherein the lymphocytes comprise T-cells and wherein the step of expanding the lymphocytes is performed for a duration until at least 60% of the T-cells express CD45RO, CD45RA, CD62L and FAS.

7. The method of any one of claims 1 to 6, wherein the expanded lymphocytes comprise memory T cells and/or do not comprise effector T cells or do comprise effector memory T cells at an amount of less than 20% of the expanded lymphocytes.

8. The method of any one of claims 1 to 7, wherein the step of expanding the lymphocytes is performed for at least 7 days, preferably for at least 10 days, even more preferred for 11-30 days.

9. The method of any one of claims 1 to 8, wherein the step of culturing the lymphocytes in a growth medium is performed for 10 to 60 hours, preferably for 12 to 48 hours.

10. The method of any one of claims 1 to 9, wherein the non-expanded lymphocytes are treated with a CD3 activator and/or a CD28 activator, preferably with an activating anti-CD3 antibody and/or an activating anti-CD28 antibody, most preferably with a colloidal polymeric nanomatrix conjugated to humanized CD3 and CD28 agonists.

11. The method of any one of claims 1 to 10, further comprising the step of inhibiting Cbl-b in the lymphocytes, preferably wherein inhibiting Cbl-b is during or after the step of expanding the lymphocytes, preferably after expanding the lymphocytes for at least 7 days.

12. The method of any one of claims 1 to 11, wherein the population of lymphocytes is a purified population of lymphocytes with at least 80% of the cell being lymphocytes, preferably with at least 80% T-cells.

13. The method of any one of claims 1 to 12, wherein the lymphocytes are CD3+ T-cells from a tumor or tumor-associated liquid of a mammal; and
culturing the lymphocytes in a growth medium comprises culturing the T-cells in a medium comprising or consisting of nutrients, a buffer, and isotonic salts, and optional stabilizers; and
expanding the lymphocytes comprises expanding the T-cells in an expansion medium comprising or consisting of nutrients, a buffer, and isotonic salts, optional stabilizers, and differentiation factors and/or cytokines consisting of interleukin-2, interleukin-7, interleukin-15, and interleukin-21.

14. A population of expanded tumor infiltrating lymphocytes (TILs) for use in a method for treating cancer, wherein the population of TILs is obtained by a method according to at least any of claims 1 to 13.

15. A population of TILs according to claim 14 for use in the treatment of cancer, especially for use in the treatment of glioblastoma.
